# EUROPEAN PATENT SPECIFICATION

(11) **EP 2 875 361 B1**
(45) Date of publication and mention of the grant of the patent: **26.12.2018**
(21) Application number: 13741705.1
(22) Date of filing: 19.07.2013
(51) Int. Cl.: G01N 33/92

(54) **GIANT LIPOSOME ARRAY FOR HIGH-THROUGHPUT LIPID-INTERACTION SCREENING**
SEHR GROSSES LIPOSOMENARRAY FÜR EIN LIPID-INTERAKTIONS-SCREENING MIT HOHEM DURCHSATZ
JEU ORDONNÉ GÉANT D'ÉCHANTILLONS DE LIPOSOMES POUR LE CRIBLAGE À HAUT DÉBIT D'INTERACTIONS AVEC LES LIPIDES

(30) Priority: 20.07.2012 GB 201212896
(43) Date of publication of application: 27.05.2015
(73) Proprietor: European Molecular Biology Laboratory (EMBL), 69117 Heidelberg (DE)
(72) Inventor: GAVIN, Anne-Claude, 69181 Leimen (DE); ELLENBERG, Jan, 69117 Heidelberg (DE); SALIBA, Antoine-Emmanuel, 97974 Heidelberg (DE); VONKOVA, Ivana, 69126 Heidelberg (DE)
(74) Representative: Zwicker, Jörk
(86) International application number: PCT/EP2013/065256
(87) International publication number: WO 2014/013043

(56) References cited:
- WO-A1-2006/102687
- WO-A2-2006/086790
- T. Osaki, et. al.: "SELECTIVE LIPID-PATTERNING FOR HETEROLOGOUS GIANT LIPOSOME ARRAY", , 2 October 2011 (2011-10-02), pages 1137-1139, XP055078616, Retrieved from the Internet: URL:www.rsc.org/images/LOC/2011/PDFs/Paper s/381_0640.pdf [retrieved on 2013-09-10]
- MARTA BALLY ET AL: "Liposome and Lipid Bilayer Arrays Towards Biosensing Applications", SMALL, vol. 6, no. 22, 22 November 2010 (2010-11-22), pages 2481-2497, XP055078236, ISSN: 1613-6810, DOI: 10.1002/smll.201000644
- TOSHIHISA OSAKI ET AL: "Towards artificial cell array system: Encapsulation and hydration technologies integrated in liposome array", MICRO ELECTRO MECHANICAL SYSTEMS (MEMS), 2012 IEEE 25TH INTERNATIONAL CONFERENCE ON, IEEE, 29 January 2012 (2012-01-29), pages 333-336, XP032137264, DOI: 10.1109/MEMSYS.2012.6170203 ISBN: 978-1-4673-0324-8
- KIM S. HORGER ET AL: "Films of Agarose Enable Rapid Formation of Giant Liposomes in Solutions of Physiologic Ionic Strength", JOURNAL OF THE AMERICAN CHEMICAL SOCIETY, vol. 131, no. 5, 11 February 2009 (2009-02-11), pages 1810-1819, XP0055078640, ISSN: 0002-7863, DOI: 10.1021/ja805625u

## Description

The present invention relates to liposome arrays, devices comprising said liposome arrays, the uses of said liposome arrays as well as methods of producing said liposome arrays.

### Background

Signalling lipids, lipid-modifying enzymes and lipid-binding proteins form a complex molecular network that regulates many cellular processes and represents an attractive target for therapeutic intervention in many diseases. Lipids are the constituents of cell membranes (Van Meer et al., 2008) but also play major role as signalling molecules. A large number of cellular processes like signal transduction and cell motility are mediated by complex lipid-protein interaction networks (Lemmon, 2008; Di Paolo & De Camilli, 2006; Groves & Kuriyan, 2010). At molecular levels, the structural determinants mediating lipid-protein recognition are still poorly understood. They imply complex interactions involving multiple lipids and protein ligands (Lemmon, 2008; Leonard et al., 2011; Gallego et al., 2010). The complexity of the binding mechanisms involved, raises the necessity of an *in vitro* assay (Groves & Kuriyan, 2010) that (i) mimics the lipid composition and biophysical state of a membrane, (ii) allows high-throughput analysis and (iii) permits quantitative analysis of the binding. Furthermore many lipid-binding proteins represent important pharmaceutical targets in a variety of life threatening diseases such as cancer and diabetes for example (Wymann & Schneiter, 2008; Carpten et al, 2007; Meuillet, 2011). A high-throughput *in vitro* assay mimicking membrane which would allow probing multiple-ligand based interactions for specific inhibitor/modulators or a combination thereof, would bypass the limits of the current methods.

WO 2006/086790 relates to a method for making a biomembrane array, comprising the preparation of a topographically patterned agarose stamp attached to a glass slide, soaking the stamp with a biomembrane solution and contacting the stamp with e.g. a glass slide to transfer the biomembrane to the glass slide, thereby forming a plurality of biomembrane film spots on the glass slide. The biomembrane film spots may comprise vesicles, liposomes, monolayer lipid membranes, etc. Osaki et al., MEMS, 2012, 333-336 relates to liposome arrays, wherein the liposomes are deposited on an agarose gel layer comprising 1% agarose in solution. Horger et al., JACS, 2009, 131, 1810-181, describes a method to form giant liposomes in solutions of physiologic ionic strength. Formation of these cell-sized liposomes proceeded from hybrid films of partially dried agarose and lipids, wherein an agarose gel layer was prepared using an aqueous solution of 1% agarose.

In contrast to other biomolecules such as nucleic acids and proteins, screening platforms for lipids are still limited by technical challenges. Currently existing methods based on protein-array (Zhu, 2001) and lipid overlay assay (Dowler, 2002) suffer from the fact that neither protein in the first case nor lipids in the second case are in a physiological conditions. Further, these platforms do not allow for quantification of the binding to catch multi-ligand interactions and necessitate a second set of *in vitro* experiments to validate obtained hits with more physiological methods based on pull-down assay or biophysical-based methods (Narayan & Lemmon, 2006; Rusten & Stenmark, 2006). Also, these latter methods cannot be scaled-up and are thus, restricted in their use. Specific assay for lipid-protein interaction inhibitor screenings based on soluble head-group lipids (Miao et al., 2010) cannot be used for disturbing protein-lipid multi-ligand interactions. On the other hand drug screening based on cell-phenotype readout (Jo et al., 2011) does not allow targeting a precise specific binding mechanism.

The present invention thus, provides a novel assay based on a liposome-array to screen lipid-protein interactions which overcomes the previously identified drawbacks associated with assays presently known in the art. The assay is based on the generation of giant liposomes on a small footprint (1 cm²) in physiological conditions opening the possibility to build a high-throughput lipid-protein assay.

The assay of the present invention allows for the generation and analysis of giant liposomes exhibiting a high encapsulation efficiency (i.e. the percent of the aqueous phase and hence the % of the water soluble drug that becomes entrapped inside the liposome during liposomes formation; or the percent of lipid soluble substances such as but not limited to proteins physiologically present in membranes or lipid soluble drugs, present in the liposome membrane). Further the generation of giant liposomes provides the advantage of a better visual analysis (Groves & Kuriyan, 2010), e.g. via microscopic means, by reducing e.g. the background noise.

In the present liposome assay any type of lipid may be used allowing for the generation of giant liposomes comprising any type or mixture of lipids. Thus, the liposome assay of the present invention allows for the analysis of giant liposomes which comprise those lipids and/or proteins also present in physiological membranes, i.e. mimicking physiological conditions as closely as possible.

A further advantage of the present liposome assay lays in the fact that only a minimal contact area between the giant liposome and the substrate exists. Thus, any disturbance of the assay itself or the achieved results is minimized.

No additional contact molecule or contact layer attaching the liposomes to the substrate layer is required in the present liposome assay. Thus, an effect caused by the presence of such contact molecule or contact layer which may e.g. impede the performance of the assay or the validity/significance of the obtained results is avoided.

Uniquely, the assay combines a high-throughput screen to a quantitative biochemical assay based on the use of fluorescence-microscopy toolbox (like FRAP or FRET). Furthermore, the assay may be integrated in a fully automated environment from the chip fabrication to result read-out: (i) cheap and fast prototyping methods are used to produce the array; (ii) lipids are dispensed automatically by commercially available spotter; (iii) the chip can be stored under inert atmosphere for long-term storage; (iv) the array can be coupled to microfluidic channels reducing reagent consumption and allowing automated fluidic control with high reproducibility and accuracy; (iv) the platform can be combined with high-content-automated microscopy; (v) automated image analysis is demonstrated. The giant liposome assay of the present invention could be produced with low-cost fabrication processes for serial production. Based on fast prototyping methods, each chip can be tuned to meet customer requirements. Primarily, the assay has two target markets: academia and pharmaceutical industry for lipid-protein interaction discovery, lipid-protein interaction quantitative study and lipid-protein interaction inhibitors research but may also be used in any other scenario wherein it may be useful.

### Summary of the Invention

This patent application provides a high-throughput, quantitative and versatile *in vitro* lipid-protein interaction assay that is designed to discover novel lipid-protein interactions and to screen chemical libraries for small-molecule that modulates these interactions. Based on fast, robust and low-cost fabrication methods combined to low reagent consumption, the assay is amenable to large-scale production, paving the way to its commercialization.

Thus, in a first aspect, the present invention provides a liposome array comprising a carrier layer, a non-continuous substrate comprising agarose on the surface of the carrier layer and one or more liposomes in contact with the non-continuous substrate, wherein the agarose has a concentration of 0.01 to 0.8 in solution.

In a second aspect, the present invention provides a liposome array comprising a carrier layer, layer of substrate comprising agarose on the surface of the carrier layer and at least two separate areas of liposomes on its surface, wherein the agarose has a concentration of 0.01 to 0.8 in solution.

In a third aspect, the present invention provides a dried liposome array comprising a liposome array according to aspects 1 or 2, wherein the solvent content in the liposome array is reduced to below 1 to 15 %.

In a fourth aspect, the present invention provides a device comprising a liposome array according to aspects 1 or 2 or a dried liposome array according to aspect 3.

In a fifth aspect, the present invention provides a method of producing the liposome array of aspects 1 or 2 comprising the steps of (a) applying a non-continuous substrate or a substrate layer to a carrier layer, preferably by rolling, spraying, deep-coating, inking, printing or microfluidic patterning, wherein the substrate comprises agarose, wherein the agarose has a concentration of 0.01 to 0.8% in solution, and (b) applying a lipid and/or a lipid composition to the non-continuous substrate or to the at least two separate areas of the substrate.

The method of the fifth aspect further comprises in a preferred embodiment the step of (c) reducing the solvent content in the liposome array to below 1-15 %.

In a sixth aspect, the present invention provides a method of studying lipid interactions comprising the step of (a) applying a sample of interest to the liposome(s) of the liposome array according to aspects 1, 2 or 3 or the device according to aspect 4.

In seventh aspect, the present invention provides a method of screening a library comprising the step of (a) applying a library to the liposome(s) of the liposome array according to aspects 1, 2 or 3 or the device according to aspect 4.

In an eighth aspect, the present invention provides a method of producing liposomes of a predetermined size comprising the steps of: (a) forming a substrate of a thickness between 1 nm to 2 µm; and (b) applying a lipid or a lipid composition to at least one surface area of the substrate.

In a ninth aspect, the present invention provides a method of aligning liposomes comprising the steps of (a) forming a non-continuous substrate; and (b) applying a lipid and/or a lipid composition to at least one surface area of the substrate; and (c) forming liposomes from the lipid and/or the lipid composition of step (b).

In a tenth aspect, the present invention provides a method of improving the shelf life of a liposome array comprising the step of: reducing the solvent content in a liposome array according to aspect 1 or 2.

### List of Figures

- **Fig. 1:**: Schematic illustration of the microfabrication technology of a liposome array without a barrier that encloses liposome areas
- **Fig. 2:**: Schematic illustration of the micro fabrication technology of a liposome array with a barrier that encloses liposome areas
- **Fig. 3:**: Schematic illustration of the micro fabrication technology of a liposome array with a barrier that encloses liposome areas which can be removed
- **Fig. 4:**: Giant liposome production (a) schematic illustration of the liposome formation and (b) phase-contrast image of liposomes formed of 1-palmitoyl-2-oleoyl-sn-glycero-3-phosphocholine (POPC) on a non-continuous substrate layer of agarose, (c) schematic illustration of the way to produce a non-continuous layer of agarose via microfluidic patterning
- **Fig. 5:**: Schematic illustration of the microchip bonding to a continuous layer of agarose
- **Fig. 6:**: Examples of micro fabrication technology (a) phase contrast image of a grid of adjacent areas of liposomes; (b) and (c) provide examples of the liposome array integrated into a microchip

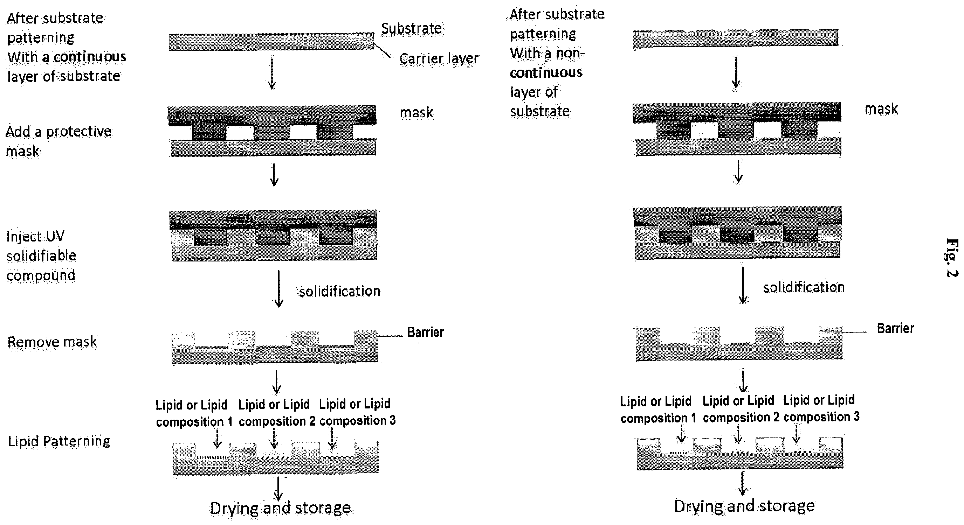

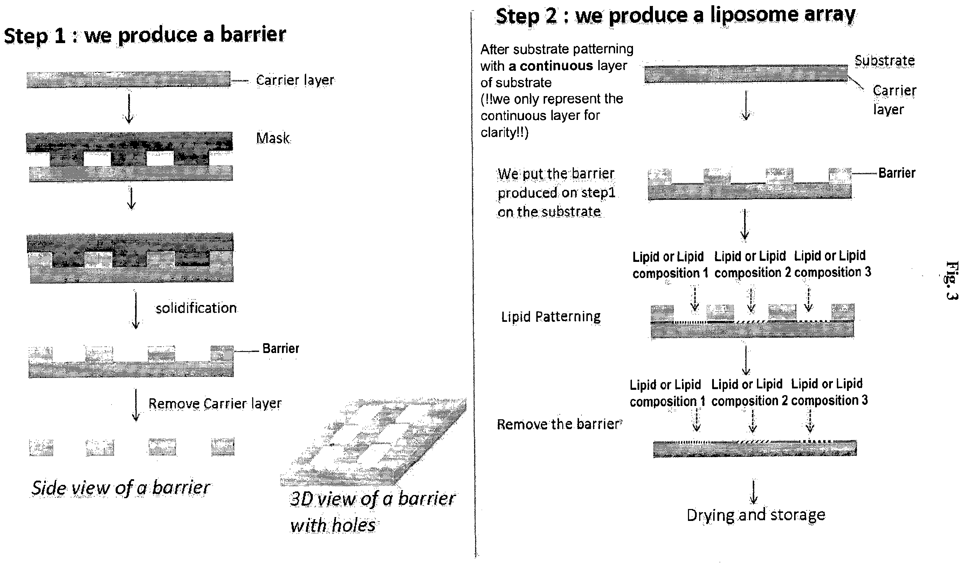

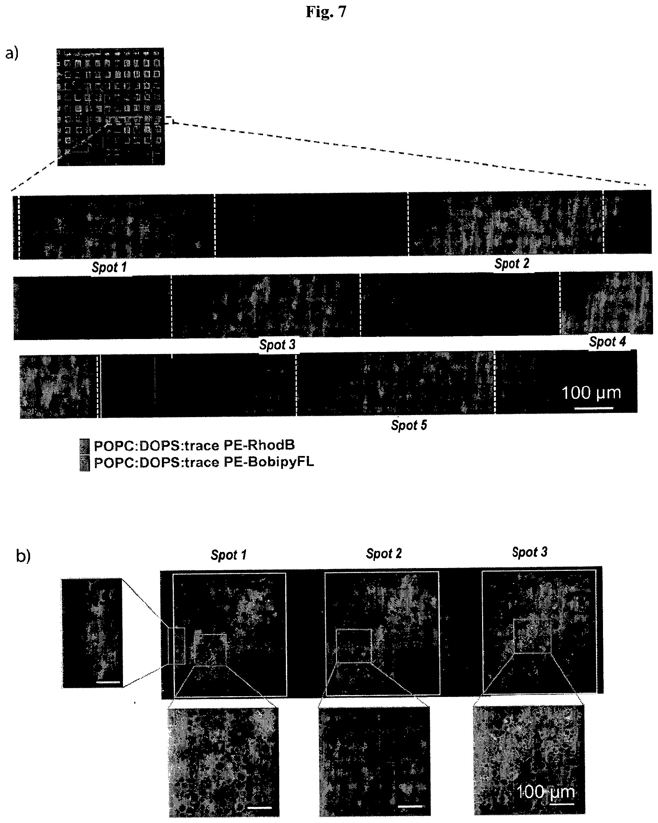

- **Fig. 7:**: Results: (a) Liposome-array formed of Rhodamin B- (spots 1, 3, 5) and Bodipy-labeled (spots 2, 4) phospholipid mixtures of POPC:DOPS. Each spots are separated by a physical barrier formed as described in procedure Fig. 2 (b) A section of liposome array, where PE BodipyFL- (spots 1 and 3) and PE-Atto647- (spot 2) containing lipid mixtures are spotted next to each other. The liposome-array is produced according to procedure described in Fig. 3 with a continuous layer of substrate. Spotting area form squares of 800x800 µm² labeled by a white square. Images are the overlay of red and green color. No cross-contamination is visible between the spotted lipid mixtures. An enlargement on the square border shows that liposomes remain within the defined spotting area. During the spotting procedure no cross-contamination occurs and once the liposomes are formed, lipids do not diffuse though the substrate.
- **Fig. 8:**: Control of liposome space and size via agarose patterning
- **Fig. 9:**: Unilamellarity of liposomes
- **Fig. 10:**: (a) Agarose layer height is proportional to the agarose concentration used to form a thin agarose layer. Each point represents an average of agarose height across 9 measurements (3 independent measurements on 3 different glass slides). (b) Determination of size of liposomes by varying the agarose concentration
- **Fig. 11:**: AKT PH domain (a) POPC only (b) POPC:DOPS, (c) POPC:PI3P, (d) POPC:PI4P, (e)POPC:PI(3,4)P2, (f) POPC:PI(3,5)P2, (g) POPC:PI(4,5)P2, (h) POPC:PI(3,4,5)P3
- **Fig. 12:**: Titration assay studying the prototypical interaction between AKT-PH domain and PI(3,4)P2 to determine the sensitivity of the assay using liposome arrays

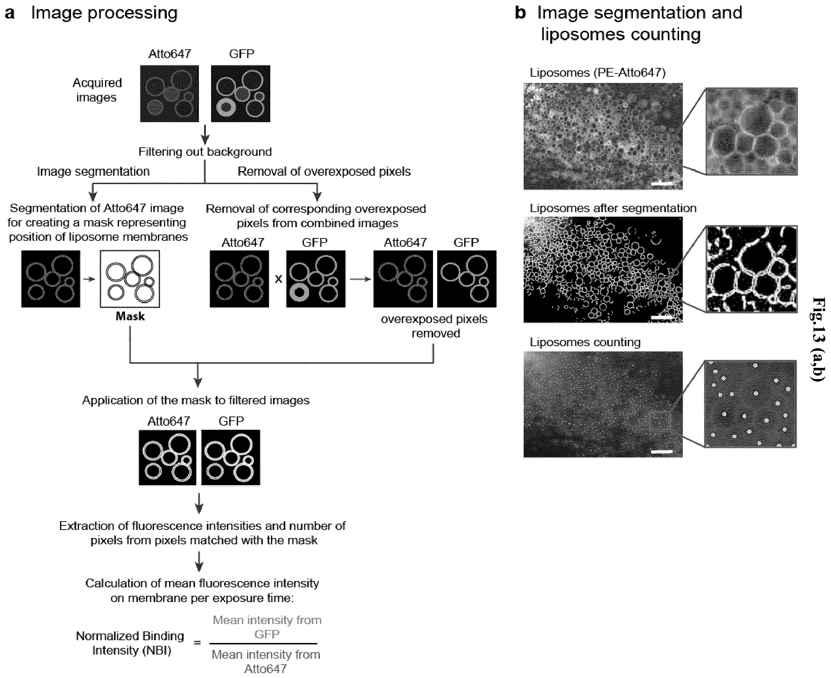

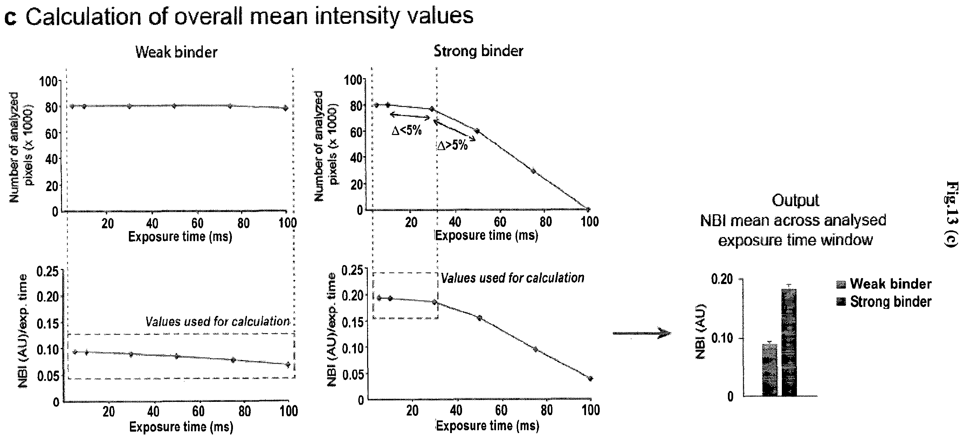

- **Fig. 13:**: High-throughput image processing and calculation of normalized binding intensity. (a) Image processing pipeline. Multiple images are acquired, one exposure time for Atto647 and multiple exposure times for GFP to capture broad range of binding intensities, from strong (short exp. times) to weak (longer exp. times). All images are then processed with CellProfiler software. After removal of background, the Atto647 image is used for segmentation to obtain a mask representing position of liposome membranes. In parallel the combination of overexposed pixels from both channels (Atto647 and GFP) is removed from every pair of Atto647 and GFP image. Subsequently, a mask is applied to all filtered (i.e. without overexposed pixels) images. Number of pixels matching with the mask and their mean fluorescence intensity is extracted from all images (i.e. both channels and every exposure time). Normalized binding intensity (NBI) is then calculated as a ratio of mean fluorescence intensity from GFP channel and mean fluorescence intensity from Atto647 channel for every pair of images. (b) Image segmentation and liposome counting. Image from Atto647 channel represents the position of liposomes. CellProfiler software is used to segment the image to retrieve a mask representing liposome membranes, and also to search for centers of liposomes that are then used for estimating a number of liposomes analyzed per image. Scale bar : 40 µm, insert of 40 µm. (c). Calculation of overall mean intensity value. To calculate overall NBI only images with no to low number of overexposed pixels are used. First, numbers of analyzed pixels (matched with the corresponding mask) per exposure time (GFP channel) are compared and all images where the drop-down of pixel number between two successive exposure times is bigger than 5% are discarded (upper plots). Next, NBI values normalized per exposure time from remaining images are used for calculation of mean NBI (lower plots). Mean NBIs of different proteins can be then compared.

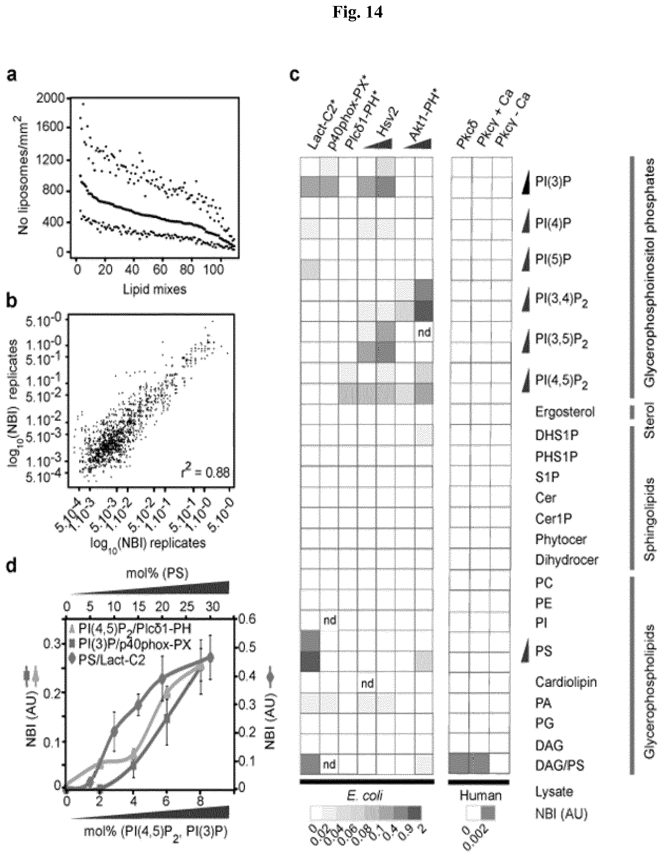

- **Fig. 14:**: Liposome Assay: quality and validation. **(a)** A thin agarose layer supports the formation of liposomes comprising a variety of different lipids. The graph shows the efficiency of liposome formation for 110 representative lipid mixtures. For each lipid mix the median of > 80 replicates are represented (middle curve). The higher and lower curves represent the quartile at 75% and 25%, respectively. Overall, the lipid mixtures tested produced a median value of 452 (s.d. 188) liposomes per mm2. (b) Reproducibility of the assay. NBIs measured for seven lipid-binding proteins (see panel c) and 30 types of liposomes were measured in biological triplicate. (c) Measurement of the binding specificity for seven lipidbinding proteins to 30 different types of liposomes. The average of three independent experiments is displayed. (*) indicates that a truncated version of the protein containing only the lipid-binding domain was tested. nd: not determined. **(d)** The liposome microarray is quantitative. The recruitment of lipid-binding domains to liposomes containing increasing amounts of the relevant signaling lipid. Error bars represent standard deviation, n = 3.

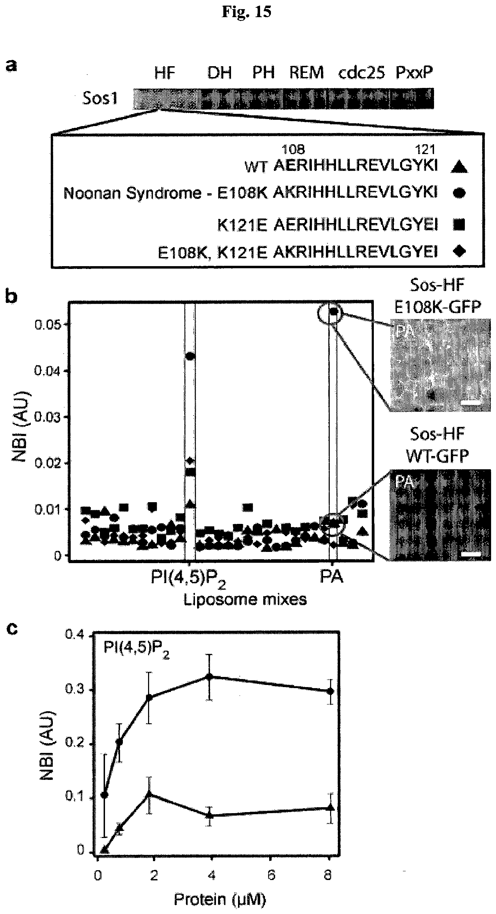

- **Fig. 15:**: An E108K mutation in Sos-HF affects its affinity for lipids. **(a)** Sos1 is a multidomain protein. Mutation of a single residue (E108K) that maps in a cluster of negatively charged amino acids (between position 108 and 121) lead to Noonan syndrome. **(b)** Sos- HF binding to liposomes of different composition. The E108K mutation leads to higher affinity for both PA and PI(4,5)P2. This phenotype is reverted by the (re)introduction of a negative charge in the binding site (K121E). c, Kinetic characterization of purified *wt* and E108K-mutated version of Sos-HF. Scale bar = 20 µm. PA, phosphatidic acid; PI(4,5)P2, phosphatidylinositol 4,5-biphosphate;Sos-HF, Son-of-sevenless histone-fold domain.
- **Fig. 16:**: Ability of a thin-agarose layer to support the formation of liposomes of various lipid compositions represented by the number of liposomes per mm²
- **Fig. 17:**: Overview of a PH domain screen. (a) PH domains and other lipid binding domains covered in the screen, and a distribution of protein concentrations used for all experiments. (b) Overview of liposome types used in the assay. (c) Summary of success rate of all experiments. (d) Evaluation of data quality based on comparison with literature-derived reference set.
- **Fig. 18:**: PH domain screen results. Overall results for the screen designed in Fig. 17 represented as a heatmap. In y and x axis, lipid mixtures and protein domains are displayed, respectively:

### Protein names (x axis)

| | | | | | | | | | |
|---|---|---|---|---|---|---|---|---|---|
| 1 | "opy1n-ph" | 12 | "osh3ct-ph" | 23 | "bud4_fused-ph" | 34 | "sytlct_b-ph" | 45 | "rtt106-ph" |
| 2 | "opy1c-ph" | 13 | "osh2-ph" | 24 | "bud4ct_2-ph" | 35 | "yell-ph" | 46 | "rtt106ct-ph" |
| 3 | "boil-ph" | 14 | "osh3-ph" | 25 | "bud4ct_fused-ph" | 36 | "yhr131c-ph" | 47 | "sec3-ph" |
| 4 | "boi2-ph" | 15 | "swh1ct-ph" | 26 | "caf120-ph" | 37 | "bem2_long-ph" | 48 | "sec3ct-ph" |
| 5 | "atg26ct-ph" | 16 | "bem3-ph" | 27 | "skg3n-ph" | 38 | "bem2_short-ph" | 49 | "num1-ph" |
| 6 | "cla4-ph" | 17 | "ysp1-ph" | 28 | "skg3c-ph" | 39 | "tus1-ph" | 50 | "ste5-ph" |
| 7 | "cla4ct-ph" | 18 | "ysplct-ph" | 29 | "ypr091c-ph" | 40 | "ynl144cct-ph" | 51 | "plcdelta-ph" |
| 8 | "skm1-ph" | 19 | "sip3-ph" | 30 | "numlct-ph" | 41 | "spo14-ph" | 52 | "caf120_2-ph" |
| 9 | "slmlct-ph" | 20 | "spo71n2-ph" | 31 | "cdc24-ph" | 42 | "spo14ct-ph" | 53 | "caf120_fused-ph" |
| 10 | "slm2-ph" | 21 | "spo71c-ph" | 32 | "syt1-ph" | 43 | "rom2-ph" | 54 | "caf120ct_fused-ph" |
| 11 | "swh1-ph" | 22 | "bud4_2-ph" | 33 | "syt1ct_a-ph" | 44 | "plclct-ph" | 55 | "eea1-fyve" |
| | | | | | | | | | |
| | | | | | | | | | |
| 56 | "eea1-fyve_short" | 67 | "rom1ct_b-ph" | 78 | "pkh2-ph" | 89 | "ira1-ph" | | |
| 57 | "p40phox-px" | 68 | "rom1ct_a-ph" | 79 | "avo1-ph" | 90 | "ira2-ph_var1" | | |
| 58 | "pleckstrin-phc" | 69 | "rom1-ph" | 80 | "exo84-ph" | 91 | "ira1-ctph" | | |
| 59 | "atg26-ph" | 70 | "plc1-ph" | 81 | "psy2-ph" | 92 | "tfb1ct-ph" | | |
| 60 | "bem3ct-ph" | 71 | "slm1-ph" | 82 | "akt1_ph" | 93 | "mdrlct-ph" | | |
| 61 | "bud4_1-ph" | 72 | "sos1-ph" | 83 | "tfb1-ph" | 94 | "exo84ct-ph" | | |
| 62 | "rgc1-ph" | 73 | "pdk1-ph" | 84 | "mdr1-ph" | 95 | "lact-c2" | | |
| 63 | "bud4ct_1-ph" | 74 | "dynamin1-ph" | 85 | "yrb2-ph" | 96 | "hsv2" | | |
| 64 | "ask10-ph" | 75 | "ira2-ph_var2" | 86 | "nup2-ph" | 97 | "ira2ct-ph" | | |
| 65 | "cdc24ct-ph" | 76 | "spo71n1-ph" | 87 | "dcp1-ph" | 98 | "ira2-CT" | | |
| 66 | "ynl44c-ph" | 77 | "caf120ct_2-ph" | 88 | "las17-ph" | 99 | "ira2ct-CT" | | |

### Lipid names (y axis)

| | | | | | |
|---|---|---|---|---|---|
| 1 | "dopi3p:sphingosine_10:10" | 43 | "dopi45p2:phyto-ceramide_10:10" | 85 | "dopi45p2:dop5_7:7" |
| 2 | "dopi3p:dhs_10:10" | 44 | "dopi45p2:di-hydro-ceramide_10:10" | 86 | "dopi3p:s-1p_10:10" |
| 3 | "dopi3p:phs-1p_10:10" | 45 | "dopi345p3:sphingosine_10:10" | 87 | "dppi4p:phs_10:10" |
| 4 | "dopi3p:dhs-1p_10:10" | 46 | "dopi345p3:phs_10:10" | 88 | "dppi4p:s-1p_10:10" |
| 5 | "dopi3p:ceramide_10:10" | 47 | "dopi345p3:dhs_10:10" | 89 | "dopi5p:phs_10:10" |
| 6 | "dopi3p:ceramide-1p_10:10" | 48 | "dopi345p3:phs-1p_10:10" | 90 | "dopi5p:phs-1p_10:10" |
| 7 | "dopi3p:phyto-ceramide_10:10" | 49 | "dopi345p3:dhs-1p_10:10" | 91 | "dopi34p2:phs-1p_10:10" |
| 8 | "dppi4p:sphingosine_10:10" | 50 | "dopi345p3:ceramide-1p_10:10" | 92 | "dopi34p2:ceramide_10:10" |
| 9 | "dppi4p:dhs_10:10" | 51 | "dopi345p3:phyto-ceramide_10:10" | 93 | "dopi34p2:di-hydro-ceramide_10:10" |
| 10 | "dppi4p:phs-1p_10:10" | 52 | "dopi345p3:di-hydro-ceramide_10:10" | 94 | "dopi35p2:sphingosine_10:10" |
| 11 | "dppi4p:dhs-1p_10:10" | 53 | "dopi3p_10" | 95 | "dopi35p2:ceramide-1p_10:10" |
| 12 | "dppi4p:ceramide_10:10" | 54 | "dppi4p_10" | 96 | "dopi35p2:di-hydro-ceramide_10:10" |
| 13 | "dppi4p:ceramide-1p_10:10" | 55 | "dopi5p_10" | 97 | "dopi345p3:s-1p_10:10" |
| 14 | "dppi4p:phyto-ceramide_10:10" | 56 | "dopi34p2_10" | 98 | "dopi35p2_10" |
| 15 | "dppi4p:di-hydro-ceramide_10:10" | 57 | "dopi45p2_10" | 99 | "di-hydro-ceramide_10" |
| 16 | "dopi5p:sphingosine_10:10" | 58 | "dopi345p3_10" | 100 | "popc_100" |
| 17 | "dopi5p:s-1p_10:10" | 59 | "sphingosine_10" | 101 | "pope:dopa_10:10:" |
| 18 | "dopi5p:dhs-1p_10:10" | 60 | "phs_10" | 102 | "dopi34p2:dops_10:10" |
| 19 | "dopi5p:ceramide_10:10" | 61 | "dhs_10" | 103 | "dag:dops_5:5" |
| 20 | "dopi5p:ceramide-1p_10:10" | 62 | "s-1p_10" | 104 | "dopi3p:di-hydro-ceramide_10:10" |
| 21 | "dopi5p:phyto-ceramide_10:10" | 63 | "phs-1p_10" | 105 | "dopi5p:dhs_10:10" |
| 22 | "dopi5p:di-hydro-ceramide_10:10" | 64 | "dhs-1p_10" | 106 | "dopi34p2:s-1p_10:10" |
| 23 | "dopi34p2:sphingosine_10:10" | 65 | "ceramide_10" | 107 | "dopi35p2:phs-1p_10:10" |
| 24 | "dopi34p2:phs_10:10" | 66 | "ceramide-1p_10" | 108 | "dopi345p3:ceramide_10:10" |
| 25 | "dopi34p2:dhs_10:10" | 67 | "phyto-ceramide_10" | 109 | "dops_10" |
| 26 | "dopi34p2:dhs-1p_10:10" | 68 | "dopi_10" | 110 | "dopi3p:phs_10:10" |
| 27 | "dopi34p2:ceramide-1p_10:10" | 69 | "cardiolipin_10" | 111 | "dppi35p2:sphingosine_10:10" |
| 28 | "dopi34p2:phyto-ceramide_10:10" | 70 | "dopg_10" | 112 | "dppi35p2:phs_10:10" |
| 29 | "dopi35p2:phs_10:10" | 71 | "pope_10" | 113 | "dppi35p2:dhs_10:10" |
| 30 | "dopi35p2:dhs_10:10" | 72 | "dopa_10" | 114 | "dppi35p2:s-1p_10:10" |
| 31 | "dopi35p2:s-1p_10:10" | 73 | "pope:dopa:dops_10:10:10" | 115 | "dppi35p2:phs-1p_10:10" |
| 32 | "dopi35p2:dhs-1p_10:10" | 74 | "pope:dopa:dops:dopi_10:10:10:10" | 116 | "dppi35p2:dhs-1p_10:10" |
| 33 | "dopi35p2:ceramide_10:10" | 75 | "dopi3p:dops_10:10" | 117 | "dppi35p2:ceramide_10:10" |
| 34 | "dopi35p2:phyto-ceramide_10:10" | 76 | "dppi4p:dops_10:10" | 118 | "dppi35p2:ceramide-1p_10:10" |
| 35 | "dopi45p2:sphingosine_10:10" | 77 | "dopi5p:dops_10:10" | 119 | "dppi35p2:phyto-ceramide_10:10" |
| 36 | "dopi45p2:phs_10:10" | 78 | "dopi35p2:dops_10:10" | 120 | "dppi35p2:di-hydro-ceramide_10:10" |
| 37 | "dopi45p2:dhs_10:10" | 79 | "dopi45p2:dops_10:10" | 121 | "dppi35p2_10" |
| 38 | "dopi45p2:s-1p_10:10" | 80 | "dopi345p3:dops_10:10" | 122 | "dppi35p2:dops_10:10" |
| 39 | "dopi45p2:phs-1p_10:10" | 81 | "dag_5" | | |
| 40 | "dopi45p2:dhs-1p_10:10" | 82 | "dhs-1p_7" | | |
| 41 | "dopi45p2:ceramide_10:10" | 83 | "dopi45p2_7" | | |
| 42 | "dopi45p2:ceramide-1p_10:10" | 84 | "dopi45p2:dhs-1p_7:7" | | |

### Detailed Description

### Definitions

Throughout this specification and the claims which follow, unless the context requires otherwise, the word "comprise", and variations such as "comprises" and "comprising", are to be understood to imply the inclusion of a stated integer or step or group of integers or steps but not the exclusion of any other integer or step or group of integer or step.

As used in this specification and the appended claims, the singular forms "a", "an", and "the" include plural referents, unless the content clearly dictates otherwise.

The term "molecule" as used herein denotes a group of at least two atoms held together by covalent bonds.

The term "lipid" as used herein denotes any fat-soluble molecule. Lipids typically consist of an aliphatic hydrocarbon chain, are poorly soluble in water but dissolve in non-polar organic solvents. Lipids are an essential group of molecules in living cells having an important role in energy storage, as structural components of cell membranes, and as signalling molecules. Examples of lipids include but are not limited to fatty acyls, fatty alcohols, sterol lipids such as cholesterol, glycerolipids such as monoglycerides (monoacylglycerol), diglycerides (diacylglycerol) or triglycerides (triacylglycerol, TAG), glycerophospholipids, saccharolipids, sphingolipids, polyketides, prenol lipids.

The term "fatty acyls" as used herein refers to a diverse group of molecules synthesized by chain elongation of an acetyl-CoA primer with malonyl-CoA (or methylmalonyl-CoA) groups that may contain a cyclic functionality and/or are substituted with heteroatoms. Examples of fatty acyls include but are not limited to fatty acids and conjugates (FA01) such as straight chain fatty acids (FA0101), branched fatty acids (FA0102), unsaturated fatty acids (FA0103), hydroperoxy fatty acids (FA0104), hydroxy fatty acids (FA0105), oxo fatty acids (FA0106), epoxy fatty acids (FA0107), methoxy fatty acids (FA0108), halogenated fatty acids (FA0109), amino fatty acids (FA0110), cyano fatty acids (FA0111), nitro fatty acids (FA0112), thia fatty acids (FA0113), carbocyclic fatty acids (FA0114), heterocyclic fatty acids (FA0115), mycolic acids (FA0116), and dicarboxylic acids (FA0117); octadecanoids (FA02) such as 12-oxophytodienoic acid metabolites (FA0201), jasmonic acids (FA0202), and other octadecanoids (FA0200); eicosanoids (FA03) such as prostaglandins (FA0301), leukotrienes (FA0302), thromboxanes (FA0303), lipoxins (FA0304), hydroxy/hydroperoxyeicosatrienoic acids (FA0305), hdroxy/hydroperoxyeicosatetraenoic acids (FA0306), hydroxy/hydroperoxyeicosapentaenoic acids (FA0307), epoxyeicosatrienoic acids (FA0308), hepoxilins (FA0309), levuglandins (FA0310), isoprostanes (FA0311), clavulones and derivatives (FA0312), and other Eicosanoids (FA0300); docosanoids (FA04); fatty alcohols (FA05); fatty aldehydes (FA06), fatty esters (FA07) such as wax monoesters (FA0701), wax diesters (FA0702), cyano esters (FA0703), lactones (FA0704), fatty acyl CoAs (FA0705), fatty acyl ACPs (FA0706), fatty acyl carnitines (FA0707), and fatty acyl adenylates (FA0708); fatty amides (FA08) such as primary amides (FA0801), N-acyl amines (FA0802), fatty acyl homoserine lactones (FA0803), and N-acyl ethanolamines (endocannabinoids) (FA0804); fatty nitriles (FA09); fatty ethers (FA10); hydrocarbons (FA11); oxygenated hydrocarbons (FA12); fatty acyl glycosides (FA13); and other fatty acyls (FA00).

The fatty acid structure is one of the most fundamental structures of biological lipids, and is commonly used as building-blocks of lipids which are structurally more complex.

"Fatty acids" are made of a hydrocarbon chain that comprises a carboxylic acid group conferring to the molecule a polar, hydrophilic head (e.g. glycerol, sphingosine), and a non-polar, hydrophobic tail that is insoluble in water. The carbon chain may be saturated or unsaturated, i.e. may comprise none, or one or more double bonds between two carbon atoms, and may have between 4 and 28 carbon atoms, i.e. 4, 5, 6, 7, 8, 9, 10, 11, 12, 13, 14, 15, 16, 17, 18, 19, 20, 21, 22, 23, 24, 25, 26, 27, or 28 carbon atoms. The double bonds in unsaturated fatty acids exist either as *cis* or as *trans* geometric isomerism affecting the molecule's molecular configuration. Further functional groups containing oxygen, halogens, nitrogen, and/or sulfur may also be attached. In living cells, fatty acids are synthesized by chain-elongation of an acetyl-CoA primer with malonyl-CoA or methylmalonyl-CoA groups through the enzyme fatty acid synthases in a process called fatty acid synthesis. Examples of saturated fatty acids include but are not limited to caprylic acid (CH3(CH2)6COOH; 8:0), capric acid (CH₃(CH₂)₈COOH; 10:0), lauric acid (CH₃(CH₂)10COOH; 12:0), myristic acid (CH₃(CH₂)₁₂COOH; 14:0), palmitic acid (CH₃(CH₂)₁₄COOH; 16:0), stearic acid (CH₃(CH₂)₁₆COOH; 18:0), arachidic acid (CH₃(CH₂)₁₈COOH; 20:0), behenic acid (CH₃(CH₂)₂₀COOH; 22:0), lignoceric acid (CH₃(CH₂)₂₂COOH; 24:0), and cerotic acid (CH₃(CH₂)₂₄COOH; 26:0).

Examples of unsaturated fatty acids include but are not limited to myristoleic acid (CH₃(CH₂)₃CH=CH(CH₂)₇COOH; 14:1), palmitoleic acid (CH₃(CH₂)₅CH=CH(CH₂)₇COOH; 16:1), sapienic acid (CH₃(CH₂)₈CH=CH(CH₂)₄COOH; 16:1), oleic acid (CH₃(CH₂)₇CH=CH(CH₂)₇COOH; 18:1), Elaidic acid (CH₃(CH₂)₇CH=CH(CH₂)₇COOH; 18:1), Vaccenic acid (CH₃(CH₂)₅CH=CH(CH₂)₉COOH; 18:1), linoleic acid (CH₃(CH₂)₄CH=CHCH₂CH=CH(CH₂)₇COOH; 18:2), Linoelaidic acid (CH₃(CH₂)₄CH=CHCH₂CH=CH(CH₂)₇COOH, 18:2), and α-linolenic acid (CH₃CH₂CH=CHCH₂CH=CHCH₂CH=CH(CH₂)₇COOH; 18:3).

The term "fatty alcohols" refers to aliphatic alcohols typically having an even number of carbon atoms (typically 8 to 36 carbon atoms) and a single alcohol group (-OH) attached to the terminal carbon.

The term "glycerolipids" refers to esterified glycerol which are mainly composed of mono-, di-, and tri-substituted glycerol with the most prominent member being the fatty acid triesters of glycerol (so-called triglycerides) wherein the three hydroxyl groups of glycerol are each esterified, typically by different fatty acids. Glycerolipids mainly function as energy storage, and thus, constitute the bulk of storage fat in animal tissues. Examples of glycerolipids include but are not limited to monoradylglycerols (GL01) such as monoacylglycerols (GL0101), monoalkylglycerols (GL0102), and mono-(1Z-alkenyl)-glycerols (GL0103); diradylglycerols (GL02) such as diacylglycerols (GL0201), 1-alkyl,2-acylglycerols (GL0202), 1-acyl,2-alkylglycerols (GL0207), dialkylglycerols (GL0203), 1Z-alkenylacylglycerols (GL0204), di-glycerol tetraethers (GL0205), and di-glycerol tetraether glycans (GL0206); triradylglycerols (GL03); glycosylmonoradylglycerols (GL04) such as glycosylmonoacylglycerols (GL0401), and glycosylmonoalkylglycerols (GL0402); glycosyldiradylglycerols (GL05) such as glycosyldiacylglycerols (GL0501), glycosylalkylacylglycerols (GL0502), and glycosyldialkylglycerols (GL0503); and other glycerolipids (GL00).

"Glycerophospholipids", herein also referred to as "phospholipids", are key components of the lipid bilayers of cells serving as a primary component of cellular membranes and binding sites for intra- and intercellular proteins. Some glycerophospholipids in eukaryotic cells, such as phosphatidylinositols and phosphatidic acids, also function as either precursors of or directly as membrane-derived second messengers. Furthermore, they are involved in metabolism and cell signalling. Glycerophospholipids comprise glycerol as alcohol to which hydroxyl groups two fatty acids and a phosphoric acid are esterified. Further, there are also alkyl-linked and 1Z-alkenyl-linked (plasmalogen) glycerophospholipids, as well as dialkylether variants in archaebacteria. Examples of glycerophospholipids include but are not limited to glycerophosphocholines (GP01) such as diacylglycerophosphocholines (GP0101), 1-alkyl,2-acylglycerophosphocholines (GP0102), 1-(1Z-alkenyl),2-acylglycerophosphocholines (GP0103), dialkylglycerophosphocholines (GP0104), monoacylglycerophosphocholines (GP0105), monoalkylglycerophosphocholines (GP0106), 1Z-alkenylglycerophosphocholines (GP0107), 1-acyl,2-alkylglycerophosphocholines (GP0108), and 1-acyl,2-(1Z-alkenyl)-glycerophosphocholines (GP0109); glycerophosphoethanolamines (GP02) such as diacylglycerophosphoethanolamines (GP0201), 1-alkyl,2-acylglycerophosphoethanolamines (GP0202), 1-(1Z-alkenyl),2-acylglycerophosphoethanolamines (GP0203), dialkylglycerophosphoethanolamines (GP0204), monoacylglycerophosphoethanolamines (GP0205), monoalkylglycerophosphoethanolamines (GP0206), 1Z-alkenylglycerophosphoethanolamines (GP0207), and 1-acyl,2-alkylglycerophosphoethanolamines (GP0208); glycerophosphoserines (GP03) such as diacylglycerophosphoserines (GP0301), 1-alkyl,2-acylglycerophosphoserines (GP0302), 1-(1Z-alkenyl),2-acylglycerophosphoserines (GP0303), dialkylglycerophosphoserines (GP0304), monoacylglycerophosphoserines (GP0305), monoalkylglycerophosphoserines (GP0306), and 1Z-alkenylglycerophosphoserines (GP0307); glycerophosphoglycerols (GP04) such as diacylglycerophosphoglycerols (GP0401), 1-alkyl,2-acylglycerophosphoglycerols (GP0402), 1-(1Z-alkenyl),2-acylglycerophosphoglycerols (GP0403), dialkylglycerophosphoglycerols (GP0404), monoacylglycerophosphoglycerols (GP0405), monoalkylglycerophosphoglycerols (GP0406), 1Z-alkenylglycerophosphoglycerols (GP0407), diacylglycerophosphodiradylglycerols (GP0408), diacylglycerophosphomonoradylglycerols (GP0409), monoacylglycerophosphomonoradylglycerols (GP0410), and 1-acyl,2-alkylglycerophosphoglycerols (GP0411); glycerophosphoglycerophosphates (GP05) such as diacylglycerophosphoglycerophosphates (GP0501), 1-alkyl,2-acylglycerophosphoglycerophosphates (GP0502), 1-(1Z-alkenyl),2-acylglycerophosphoglycerophosphates (GP0503), dialkylglycerophosphoglycerophosphates (GP0504), monoacylglycerophosphoglycerophosphates (GP0505), monoalkylglycerophosphoglycerophosphates (GP0506), and 1Z-alkenylglycerophosphoglycerophosphates (GP0507); Glycerophosphoinositols (GP06) such as diacylglycerophosphoinositols (GP0601), 1-alkyl,2-acylglycerophosphoinositols (GP0602), 1-(1Z-alkenyl),2-acylglycerophosphoinositols (GP0603), dialkylglycerophosphoinositols (GP0604), monoacylglycerophosphoinositols (GP0605), monoalkylglycerophosphoinositols (GP0606), and 1Z-alkenylglycerophosphoinositols (GP0607); glycerophosphoinositol monophosphates (GP07) such as diacylglycerophosphoinositol monophosphates (GP0701), 1-alkyl,2-acylglycerophosphoinositol monophosphates (GP0702), 1-(1Z-alkenyl),2-acylglycerophosphoinositol monophosphates (GP0703), dialkylglycerophosphoinositol monophosphates (GP0704), monoacylglycerophosphoinositol monophosphates (GP0705), monoalkylglycerophosphoinositol monophosphates (GP0706), and 1Z-alkenylglycerophosphoinositol monophosphates (GP0707); glycerophosphoinositol bisphosphates (GP08) such as diacylglycerophosphoinositol bisphosphates (GP0801), 1-alkyl,2-acylglycerophosphoinositol bisphosphates (GP0802), 1-(1Z-alkenyl),2-acylglycerophosphoinositol bisphosphates (GP0803), monoacylglycerophosphoinositol bisphosphates (GP0804), monoalkylglycerophosphoinositol bisphosphates (GP0805), and 1Z-alkenylglycerophosphoinositol bisphosphates (GP0806); glycerophosphoinositol trisphosphates (GP09) such as diacylglycerophosphoinositol trisphosphates (GP0901), 1-alkyl,2-acylglycerophosphoinositol trisphosphates (GP0902), 1-(1Z-alkenyl),2-acylglycerophosphoinositol trisphosphates (GP0903), monoacylglycerophosphoinositol trisphosphates (GP0904), monoalkylglycerophosphoinositol trisphosphates (GP0905), and 1Z-alkenylglycerophosphoinositol trisphosphates (GP0906); glycerophosphates (GP10) such as diacylglycerophosphates (GP1001), 1-alkyl,2-acylglycerophosphates (GP1002), 1-(1Z-alkenyl),2-acylglycerophosphates (GP1003), dialkylglycerophosphates (GP1004), monoacylglycerophosphates (GP1005), monoalkylglycerophosphates (GP1006), and 1Z-alkenylglycerophosphates (GP1007); Glyceropyrophosphates (GP11) such as diacylglyceropyrophosphates (GP1101) and monoacylglyceropyrophosphates (GP1102); glycerophosphoglycerophosphoglycerols (GP12) such as diacylglycerophosphoglycerophosphodiradylglycerols (GP 1201), diacylglycerophosphoglycerophosphomonoradylglycerols (GP 1202), 1-alkyl,2-acylglycerophosphoglycerophosphodiradylglycerols (GP 1203), 1-alkyl,2-acylglycerophosphoglycerophosphomonoradylglycerols (GP 1204), 1-(1Z-alkenyl),2-acylglycerophosphoglycerophosphodiradylglycerols (GP 1205), 1-(1Z-alkenyl),2-acylglycerophosphoglycerophosphomonoradylglycerols (GP 1206), monoacylglycerophosphoglycerophosphomonoradylglycerols (GP 1207), monoalkylglycerophosphoglycerophosphodiradylglycerols (GP1208), monoalkylglycerophosphoglycerophosphomonoradylglycerols (GP 1209), 1Z-alkenylglycerophosphoglycerophosphodiradylglycerols (GP 1210), 1Z-alkenylglycerophosphoglycerophosphomonoradylglycerols (GP 1211), dialkylglycerophosphoglycerophosphodiradylglycerols (GP 1212) and dialkylglycerophosphoglycerophosphomonoradylglycerols (GP1213); CDP-Glycerols (GP13) such as CDP-diacylglycerols (GP1301), CDP-1-alkyl,2-acylglycerols (GP1302), CDP-1-(1Z-alkenyl),2-acylglycerols (GP1303), CDP-dialkylglycerols (GP1304), CDP-monoacylglycerols (GP1305), CDP-monoalkylglycerols (GP1306), and CDP-1Z-alkenylglycerols (GP1307); glycosylglycerophospholipids (GP14) such as diacylglycosylglycerophospholipids (GP1401), 1-alkyl,2-acylglycosylglycerophospholipids (GP 1402), 1-(1Z-alkenyl),2-acylglycosylglycerophospholipids (GP1403), monoacylglycosylglycerophospholipids (GP1404), monoalkylglycosylglycerophospholipids (GP1405), 1Z-alkenylglycosylglycerophospholipids (GP1406), and dialkylglycosylglycerophospholipids (GP1407); glycerophosphoinositolglycans (GP15) such as diacylglycerophosphoinositolglycans (GP 1501), 1-alkyl,2-acylglycerophosphoinositolglycans (GP1502), 1-(1Z-alkenyl),2-acylglycerophosphoinositolglycans (GP1503), monoacylglycerophosphoinositolglycans (GP 1504), monoalkylglycerophosphoinositolglycans (GP1505), 1Z-alkenylglycerophosphoinositolglycans (GP1506), and dialkylglycerophosphoinositolglycans (GP1507); glycerophosphonocholines (GP16) such as diacylglycerophosphonocholines (GP1601), 1-alkyl,2-acylglycerophosphonocholines (GP1602), 1-(1Z-alkenyl),2-acylglycerophosphonocholines (GP1603), dialkylglycerophosphonocholines (GP1604), monoacylglycerophosphonocholines (GP 1605), monoalkylglycerophosphonocholines (GP1606), and 1Z-alkenylglycerophosphonocholines (GP1607); glycerophosphonoethanolamines (GP17) such as diacylglycerophosphonoethanolamines (GP1701), 1-alkyl,2-acylglycerophosphonoethanolamines (GP1702), 1-(1Z-alkenyl),2-acylglycerophosphonoethanolamines (GP 1703), dialkylglycerophosphonoethanolamines (GP1704), monoacylglycerophosphonoethanolamines (GP1705), monoalkylglycerophosphonoethanolamines (GP1706), and 1Z-alkenylglycerophosphonoethanolamines (GP1707); di-glycerol tetraether phospholipids (caldarchaeols) (GP18); glycerol-nonitol tetraether phospholipids (GP19); oxidized glycerophospholipids (GP20) such as oxidized glycerophosphocholines (GP2001), oxidized glycerophosphoethanolamines (GP2002), and oxidized Cardiolipins (GP2003); and other Glycerophospholipids (GP00).

In biological membranes glycerophospholipids including but not limited to phosphatidic acid (phosphatidate) (PA), phosphatidylethanolamine (cephalin) (PE), phosphatidylcholine (lecithin) (PC), phosphatidylserine (PS), and phosphoinositides such as phosphatidylinositol (PI), phosphatidylinositol phosphate (PIP), phosphatidylinositol bisphosphate (PIP2), phosphatidylinositol triphosphate (PIP3), cardiolipin and lysophospholipids, are most prominent.

Naturally occurring phospholipid derivates include but are not limited to egg PC, egg PG, soy PC, hydrogenated soy PC, and sphingomyelin. Synthetic phospholipid derivates include but are not limited to phosphatidic acid (DMPA, DPPA, DSPA), phosphatidylcholine (DDPC, DLPC, DMPC, DPPC, DSPC, DOPC, POPC, DEPC), phosphatidylglycerol (DMPG, DPPG, DSPG, POPG), phosphatidylethanolamine (DMPE, DPPE, DSPE DOPE), phosphatidylserine (DOPS), and PEG phospholipid (mPEG-phospholipid, polyglycerin-phospholipid, funcitionalized-phospholipid, terminal activated-phospholipid)

"Sphingolipids" comprise a sphingoid base backbone with the major sphingoid base of mammals being sphingosine. The main mammalian sphingoid bases are dihydrosphingosine and sphingosine, while dihydrosphingosine and phytosphingosine are the principle sphingoid bases in yeast. The sphingosine backbone may be O-linked to a typically charged head group such as ethanolamine, serine, or choline, and amide-linked to an acyl group, such as a fatty acid. The fatty acids are typically saturated or mono-unsaturated with chain lengths from 16 to 26 carbon atoms. Examples of sphingolipids include but are not limited to sphingoid bases (SP01) such as sphing-4-enines (Sphingosines) (SP0101), sphinganines (SP0102), 4-Hydroxysphinganines (Phytosphingosines) (SP0103), Sphingoid base homologs and variants (SP0104), Sphingoid base 1-phosphates (SP0105), Lysosphingomyelins and lysoglycosphingolipids (SP0106), N-methylated sphingoid bases (SP0107), and Sphingoid base analogs (SP0108); ceramides (SP02) such as N-acylsphingosines (ceramides) (SP0201), N-acylsphinganines (dihydroceramides) (SP0202), N-acyl-4-hydroxysphinganines (phytoceramides) (SP0203), acylceramides (SP0204), and ceramide 1-phosphates (SP0205); phosphosphingolipids (SP03) such as ceramide phosphocholines (sphingomyelins) (SP0301), ceramide phosphoethanolamines (SP0302), and ceramide phosphoinositols (SP0303); phosphonosphingolipids (SP04); neutral glycosphingolipids (SP05) such as simple Glc series (SP0501), GalNAcβ1-3Galα1-4Galβ1-4Glc- (Globo series) (SP0502), GalNAcβ1-4Galβ1-4Glc- (Ganglio series) (SP0503), Galβ1-3GlcNAcβ1-3Galβ1-4Glc- (Lacto series) (SP0504), Galβ1-4GlcNAcβ1-3Galβ1-4Glc- (Neolacto series) (SP0505), GalNAcβ1-3Galα1-3Galβ1-4Glc- (Isoglobo series) (SP0506), GlcNAcβ1-2Manα1-3Manβ1-4Glc- (Mollu series) (SP0507), GalNAcβ1-4GlcNAcβ1-3Manβ1-4Glc- (Arthro series) (SP0508), Gal- (Gala series) (SP0509) and other neutral glycosphingolipids (SP0500); acidic glycosphingolipids (SP06) such as gangliosides (SP0601), sulfoglycosphingolipids (sulfatides) (SP0602), glucuronosphingolipids (SP0603), phosphoglycosphingolipids (SP0604), and other acidic glycosphingolipids (SP0600); basic glycosphingolipids (SP07); amphoteric glycosphingolipids (SP08); arsenosphingolipids (SP09); and other sphingolipids (SP00).

Ceramides (N-acyl-sphingoid bases) are a major subclass of sphingoid base derivatives with amide-linked fatty acids. Biologically relevant examples of ceramides include but are not limited to ceramide phosphorylcholine (SPH), ceramide phosphorylethanolamine (Cer-PE), and ceramide phosphorylglycerol. The major phosphosphingolipids of mammals are sphingomyelins (e.g. ceramide, phosphocholines). "Glycosphingolipids" are composed of one or more sugar residues linked via a glycosidic bond to the sphingoid base. Examples are glycosphingolipids such as but not limited to cerebrosides and gangliosides.

The term "saccharolipids" refers to molecules in which fatty acids are linked directly to a sugar backbone, forming structures that are compatible with membrane bilayers. Examples of saccharolipids include but are not limited to acylaminosugars (SL01), such as monoacylaminosugars (SL0101), diacylaminosugars (SL0102), triacylaminosugars (SL0103), tetraacylaminosugars (SL0104), pentaacylaminosugars (SL0105), hexaacylaminosugars (SL0106), and heptaacylaminosugars (SL0107); acylaminosugar glycans (SL02); acyltrehaloses (SL03); acyltrehalose glycans (SL04); other acyl sugars (SL05); and other saccharolipids (SL00).

Along with glycerophospholipids and sphingolipids "sterol lipids", also called "steroids", such as but not limited to cholesterol and its derivatives, are important components of cellular membrane lipids playing various biological roles as hormones and as signaling molecules. Steroids have a core structure of four fused carbon rings which may be esterified to a carbon chain. The eighteen-carbon (C18) steroids include the estrogen family whereas the C19 steroids comprise the androgens such as testosterone and androsterone. The C21 subclass includes the progestogens as well as the glucocorticoids and mineralocorticoids. The secosteroids, comprising various forms of vitamin D, are characterized by cleavage of the B ring of the core structure. Other examples of sterols are bile acids and their conjugates, which in mammals are oxidized derivatives of cholesterol and are synthesized in the liver. The plant equivalents are the phytosterols, such as β-sitosterol, stigmasterol, and brassicasterol; the latter compound is also used as a biomarker for algal growth. The predominant sterol in fungal cell membranes is ergosterol. Examples of sterol lipids include but are not limited to sterols (ST01) such as cholesterol and derivatives (ST0101), cholesteryl esters (ST0102), ergosterols and C24-methyl derivatives (ST0103), stigmasterols and C24-ethyl derivatives (ST0104), C24-propyl sterols and derivatives (ST0105), gorgosterols and derivatives (ST0106), furostanols and derivatives (ST0107), spirostanols and derivatives (ST0108), furospirostanols and derivatives (ST0109), cycloartanols and derivatives (ST0110), calysterols and cyclopropyl sidechain derivatives (ST0111), cardanolides and derivatives (ST0112), bufanolides and derivatives (ST0113), brassinolides and derivatives (ST0114), solanidines and alkaloid derivatives (ST0115), and withanolides and derivatives (ST0116); steroids (ST02) such as C18 steroids (estrogens) and derivatives (ST0201), C19 steroids (androgens) and derivatives (ST0202), and C21 steroids (gluco/mineralocorticoids, progestogins) and derivatives (ST0203); secosteroids (ST03) such as vitamin D2 and derivatives (ST0301), vitamin D3 and derivatives (ST0302), vitamin D4 and derivatives (ST0303), vitamin D5 and derivatives (ST0304), vitamin D6 and derivatives (ST0305), and vitamin D7 and derivatives (ST0306); Bile acids and derivatives (ST04) such as C24 bile acids, alcohols, and derivatives (ST0401), C26 bile acids, alcohols, and derivatives (ST0402), C27 bile acids, alcohols, and derivatives (ST0403), C28 bile acids, alcohols, and derivatives (ST0404), C22 bile acids, alcohols, and derivatives (ST0405), C23 bile acids, alcohols, and derivatives (ST0406), C25 bile acids, alcohols, and derivatives (ST0407), and C29 bile acids, alcohols, and derivatives (ST0408); Steroid conjugates (ST05) such as glucuronides (ST0501), sulfates (ST0502), glycine conjugates (ST0503), taurine conjugates (ST0504) and other Steroid conjugates (ST0505); and other sterol lipids (ST00).

The term "polyketides" as used herein refers to a family comprising structurally very diverse members all of which are synthesized via the polyketide synthase pathway through the decarboxylative condensation of malonyl-CoA derived extender units in a similar process to fatty acid synthesis. Polyketides are broadly divided into three classes: type I polyketides (typically macrolides produced by multimodular megasynthases), type II polyketides (typically aromatic molecules produced by the iterative action of dissociated enzymes), and type III polyketides (typically small aromatic molecules produced by fungal species). Commercially used polyketide include natural antibiotics, antifungals, cytostatics, anticholesteremic, antiparasitics, coccidiostats, animal growth promoters and insecticides. Examples of polyketides include but are not limited to linear polyketides (PK01); halogenated acetogenins (PK02); annonaceae acetogenins (PK03); macrolides and lactone polyketides (PK04); ansamycins and related polyketides (PK05); polyenes (PK06); linear tetracyclines (PK07); angucyclines (PK08); polyether polyketides (PK09); aflatoxins and related substances (PK10); cytochalasins (PK11); flavonoids (PK12) such as anthocyanidins (PK1201), flavans, flavanols and leucoanthocyanidins (PK1202), proanthocyanidins (PK1203), biflavonoids and polyflavonoids (PK1204), isoflavonoids (PK1205), rotenoid flavonoids (PK1206), pterocarpans (PK1207), isoflavans (PK1208), coumestan flavonoids (PK1209), neoflavonoids (PK1210), flavones and flavonols (PK1211), chalcones and dihydrochalcones (PK1212), aurone flavonoids (PK1213), flavanones (PK1214), dihydroflavonols (PK1215), and other flavonoids (PK1216); aromatic polyketides (PK13) such as monocyclic aromatic polyketides (PK1301), naphthalenes and naphthoquinones (PK1302), benzoisochromanquinones (PK1303), anthracenes and phenanthrenes (PK1304), anthracyclinones (PK1305), dibenzofurans, griseofulvins, dibenzopyrans and xanthones (PK1306), diphenylmethanes, acylphloroglucinols and benzophenones (PK1307), depsides and depsidones (PK1308), diphenyl ethers, biphenyls, dibenzyls and stilbenes (PK1309), benzofuranoids (PK1310), benzopyranoids (PK1311), and other aromatic polyketides (PK1312); non-ribosomal peptide/polyketide hybrids (PK14); and other polyketides (PK00).

The term "prenol lipids" refers to molecules synthesized from the five carbon precursors isopentenyl diphosphate and dimethylallyl diphosphate and are mainly produced via the mevalonic acid pathway. In some bacteria (e.g. Escherichia coli) and plants, isoprenoid precursors are made via the methylerythritol phosphate pathway. Because simple isoprenoids (linear alcohols, diphosphates, etc.) are formed by the successive addition of C5 units, isoprenoids are conveniently classified accordingly, with a polyterpene subclass for those structures containing more than 40 carbons (i.e., 8 isoprenoid units). Prenol lipids and their phosphorylated derivatives play important roles in the transport of oligosaccharides across membranes. Polyprenol phosphate sugars and polyprenol diphosphate sugars function in extracytoplasmic glycosylation reactions, in extracellular polysaccharide biosynthesis (for instance, peptidoglycan polymerization in bacteria), and in eukaryotic protein N-glycosylation. Examples of prenol lipids include but are not limited to isoprenoids (PR01) such as C5 isoprenoids (hemiterpenes) (PR0101), C10 isoprenoids (monoterpenes) (PR0102), C15 isoprenoids (sesquiterpenes) (PR0103), C20 isoprenoids (diterpenes) (PR0104), C25 isoprenoids (sesterterpenes) (PR0105), C30 isoprenoids (triterpenes) (PR0106), C40 isoprenoids (tetraterpenes) (PR0107), polyterpenes (PR0108), and retinoids (PR0109); quinones and hydroquinones (PR02) such as ubiquinones (PR0201), vitamin E (PR0202), and vitamin K (PR0203); polyprenols (PR03) such as bactoprenols (PR0301), bactoprenol monophosphates (PR0302), bactoprenol diphosphates (PR0303), phytoprenols (PR0304), phytoprenol monophosphates (PR0305), phytoprenol diphosphates (PR0306), dolichols (PR0307), dolichol monophosphates (PR0308), and dolichol diphosphates (PR0309); hopanoids (PR04); and other prenol lipids (PR00).

The term "lipid bilayer" as used herein refers to a double layer structure of lipids, typically spontaneously formed in aqueous environments, wherein the hydrophilic heads face the water at each surface of the bilayer, and the hydrophobic tails are shielded from the water in the interior. As used herein, the term encompasses bilayers of all geometries including but not limited to planar and curved bilayers.

The term "micelle" as used herein refers to an aggregate of lipids dispersed in a liquid colloid. A typical micelle in aqueous solution forms an aggregate wherein the hydrophilic heads are in contact with the surrounding solution and the hydrophobic tails are in the center of the micelle. In a non-polar solvent, certain lipids may also form inverted micelles.

The term "vesicle" as used herein refers to a small closed cavity enclosing a liquid. Typically, a vesicle has a size of below 500 µm i.e. below 1, 2, 3, 4, 5, 10, 15, 20, 25, 50, 75, 100, 150, 200, 250, 300, 350, 400, 450, or 500 µm.

The term "liposome" as used throughout the description and the claims refers to a vesicle comprising a lipid bilayer membrane. Thus, liposomes differ from micelles in that they comprise a lipid bilayer whereas micelles are composed of lipid monolayers. The lipid membrane of the liposome may comprise components such as but not limited to lipids, proteins, and other membrane-associated components. The major types of liposomes include multilamellar vesicle (MLV), small unilamellar vesicle (SUV), large unilamellar vesicle (LUV), giant unilamellar vesicle (GUV). Typically, the diameter of SUV and LUV liposomes is between 1 nm and 1 µm and the diameter of GUV liposomes is between 1 µm and 300 µm, i.e. 1, 2, 3, 4, 5, 10, 15, 20, 25, 50, 75, 100, 150, 200, 250, or 300 µm. Typically, liposomes encapsulate a liquid core, often an aqueous solution, inside the hydrophobic membrane. Whilst dissolved hydrophilic solutes cannot readily pass through the lipid bilayer, hydrophobic chemicals can be dissolved into the membrane, thus, a liposome may comprise both hydrophobic and hydrophilic molecules. The lipid bilayer of a liposome may fuse with other bilayers such as other liposomes or cell membranes. Thereby liposomes may deliver their contents to the second liposome or to the cell. Thus, to avoid the fusion of two neighboring liposomes, these liposomes are typically kept at a certain distance from each other to prevent the fusion of the lipid bilayers. The extend of the distance between two liposomes depends on the size of the liposomes, the composition of liposomes and the chemical nature of the solvent. Commercially, liposomes are often made in a solution of DNA or drugs (which would normally not be able to diffuse through a membrane) such that the liposome encapsulates said DNA or drugs which may then be delivered past the lipid bilayer, e.g. of the cell of a patient. Liposomes are also used for the transformation or transfection of DNA into a host cell in a process known as lipofection. In addition to drug and gene delivery applications, liposomes may also be used to deliver pesticides to plants, enzymes and nutritional supplements to foods, and cosmetics to the skin, and even of dyes to textiles. Liposomes may also be used as outer shells of some microbubble contrast agents used in contrast-enhanced ultrasound.

The term "area of liposomes" as used herein refers to an area, vicinity or spot which comprises any number of liposomes. Optionally, an area of liposomes is enclosed such that no exchange of liposomes occurs between adjacent areas or adjacent areas of liposomes. The enclosure may be obtained by any means functioning as "separating barrier" such as but not limited to a barrier consisting of a material selected from the group consisting of a polymer, glass, ceramic, and plastic. Polymers may be UV and/or heat sensitive polymers, such as but not limited to thiolene based resin, ceramic, poly-(methyl methacrylate), and cyclic olefin copolymer.

The term "carrier" as used herein refers to any means upon which further material may be placed or mounted. A "carrier layer" thus, refers to a layer functioning as a carrier upon which further layers may be placed or mounted. Depending on the required use, the carrier or carrier layer may be transparent or opaque and may be produced from any suitable material including but not limited to glass, silicon, polymer, ceramic, and plastic.

The term "substrate" as used herein refers to any material suitable to serve as a basis whereupon liposomes may be placed, preferably a material liposomes attach to and/or are formed upon. The substrate may be any kind of material suitable, such as e.g. an organic substrate, including but not limited to organic or hybrid polymers, or a non-conductive substrate that has a resistance of more than 100 ohms, more than 1000 ohms, or even more than 10 000 ohms. Preferably, the substrate is an organic polymer, more preferable a hydrogel. Hydrogels (also called aquagel) consist of a network of hydrophilic, highly absorbent (they can contain over 99.9% water) natural or synthetic polymer chains. Substrates may comprise "polymerizing agent" which form polymer chains or three-dimensional networks by chemically reacting monomer molecules step-wise or chain-wise together. Step-growth polymers are polymers formed by the stepwise reaction between functional groups of monomers, usually containing heteroatoms such as nitrogen or oxygen, whereas chain-growth polymerization (or addition polymerization) involves the linking together of molecules incorporating double or triple carbon-carbon bonds. Examples of commonly used polymerizing agents include but are not limited to agarose, acrylate, polyether polymer, gelatine, acrylamide and matrigel. In the context of the present invention the substrate is agarose.

The substrate may be continuous or non-continuous. The term "continuous substrate" as used herein refers to an unbroken layer of substrate, i.e. which is not interrupted by any other material. The term "non-continuous substrate" as used herein refers to substrate shaped in a particular pattern wherein areas of substrate are interchanged with areas of a different material, e.g. with areas of a carrier or carrier layer. Non-Continuous substrate may be patterned in a shape including but not limited to stripes, ridges, squares, rectangular, circles, and spirals.

The term "solvent" as used herein refers to any liquid, solid, or gas that dissolves another solid, liquid, or gaseous solute, resulting in a solution that is soluble in a certain volume of solvent at a specified temperature. Examples of non-polar solvents include but are not limited to pentane, cyclopentane, hexane, cyclohexane, benzene, toluene, 1,4-dioxane, chloroform, and diethylether. Examples of polar aprotic solvents include but are not limited to dichloromethane (DCM), tetrahydrofuran (THF), ethyl acetate, acetone, dimethylformamide (DMF), acetonitrile (MeCN), dimethyl sulfoxide (DMSO), propylene carbonate. Examples of polar protic solvents include but are not limited to water, acetic acid, methanol, ethanol, n-propanol, isopropanol, n-butanol, and formic acid. Commonly used organic solvents include but are not limited to ethanol, acetone, methyl acetate, ethyl acetat, toluene, terpenes, turpentine, tetrachloroethylene, hexane, petrol ether, and DMSO. Typically lipids are dissolved in any chloroform-based solvent. The term "solvent" also includes physiological buffers such as but not limited to phosphate buffered saline (PBS), 4-2-hydroxyethyl-1-piperazineethanesulfonic acid (HEPES), potassium based buffer (KCl), tris(hydroxymethyl)methylamine (TRIS), 2-(N-morpholino)ethanesulfonic acid (MES), 3-(N-morpholino)propanesulfonic acid (MOPS), piperazine-N,N'-bis(2-ethanesulfonic acid) (PIPES), saline sodium citrate (SSC), N-tris(hydroxymethyl)methylglycine (tricine), 3-{[tris(hydroxymethyl)methyl]amino}propanesulfonic acid (TAPS), N,N-bis(2-hydroxyethyl)glycine (bicine), 3-[N-Tris(hydroxymethyl)methylamino]-2-hydroxypropanesulfonic Acid (TAPSO), 2-{[tris(hydroxymethyl)methyl]amino} ethanesulfonic acid (TES), and dimethylarsinic acid (cacodylate).

The terms "microchip" and "microfluidic chip" are used interchangeably herein, referring to a two-dimensional array mounted on a solid carrier (typically a glass slide or silicon thin-film cell) which allows assaying large amounts of biological material using high-throughput screening methods. Often a panel of related tests is analyzed simultaneously in a single sample using well-known methods such as but not limited to sandwich, competitive and antibody-capture immunoassays. Microfluidic chips are typically produced in that in a first step open channels are formed in a mask or mold which may be made of any material suitable including but not limited to glass, ceramic, plastic or any polymer such as e.g. PDMS. In a second step, the mask or mold is attached to a carrier or carrier layer such that microfluidic channels are formed between the open channels of the mold and the carrier. The construction of microfluidic chips is further described in detail in Wu, Hongkai, et al. (2005) Lab Chip, Vol. 5, pages 1393-1398. Typically, a microchip comprises means for the application of fluid. Examples of according means include but are not limited to microfluidic means such as microchannels and micropumps. Devises comprising one or more microchips may further comprise additional components including but not limited to seringe pumps, perisaltic pumps, and pressure driven pumps.

### Embodiments

In a first aspect, the present invention provides a liposome array allowing analyzing lipid interaction with other molecules, such as but not limited to proteins, nucleic acids or chemical compounds such as e.g. pharmaceutically active compounds. Said liposome array comprises a carrier layer, a non-continuous substrate on the surface of the carrier layer and one or more liposomes in contact with the non-continuous substrate. The carrier layer may be either transparent or opaque. In preferred embodiments of the first aspect of the present invention the carrier layer is transparent. The carrier may be made of any suitable material, preferably selected from the group consisting of glass, silicon, polymer, ceramic, and plastic. In particularly preferred embodiments, the carrier layer is made of glass.

In preferred embodiments all or a part of the non-continuous substrate is patterned. Preferably, the pattern has the shape selected from the group consisting of stripes, squares, rectangles, spots, circles and spirals. Typically, the pattern of the non-continuous substrate determines the position of the liposomes and the distance the liposomes have from each other. In preferred embodiments the non-continuous substrate is in the shape of one or more linear or curved stripes or ridges. These stripes or ridges preferably have a width of less than 10,000 µm, i.e. less than 10,000 µm, less than 9,000 µm, less than 8,000 µm, less than 7,000 µm, less than 6,000 µm, less than 5,000 µm, less than 4,000 µm, less than 3,000 µm, less than 2,000 µm, less than, 1,000 µm, less than 900 µm, less than 800 µm, less than 700 µm, less than 600 µm, less than 500 µm, less than 400 µm, less than 300 µm, less than 200 µm, less than 100 µm, less than 99 µm, less than 98 µm, less than 97 µm, less than 96 µm, less than 95 µm, less than 94 µm, less than 93 µm, less than 92 µm, less than 91 µm, less than 90 µm, less than 85 µm, less than 80 µm, less than 75 µm, less than 70 µm, less than 65 µm, less than 60 µm, less than 55 µm, less than 50 µm, less than 45 µm, less than 40 µm, less than 35 µm, less than 30 µm, less than 25 µm, less than 20 µm, less than 15 µm, less than 10 µm, less than 5 µm, less than 4 µm, less than 3 µm, less than 2 µm, less than 1 µm, or less than 0.5 µm. It is particularly preferred that the width of stripes or ridges is 1 µm to 100 µm, more preferably about 10 µm.

In further preferred embodiments, the distance between adjacent stripes or ridges is 0.1 µm or more, i.e. 0.1 µm, 0.2 µm, 0.3 µm, 0.4 µm, 0.5 µm, 0.6 µm, 0.7 µm, 0.8 µm, 0.9 µm, 1 µm, 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, 20 µm, 25 µm, 30 µm, 35 µm, 40 µm, 45 µm, 50 µm, 55 µm, 60 µm, 70 µm, 75 µm, 80 µm, 85 µm, 90 µm, 95 µm, 100 µm, or more. It is particularly preferred that the distance between adjacent stripes or ridges is between 5 µm and 15 µm, most preferably about 10 µm.

Preferably, the thickness of the substrate is between 1 nm to 2 µm, i.e. 1 nm, 2 nm, 3 nm, 4 nm, 5 nm, 6 nm, 7 nm, 8 nm, 9 nm, 10 nm, 15 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 200 nm, 300 nm, 400 nm, 500 nm, 600 nm, 700 nm, 800 nm, 900 nm, 1 µm, 1.1 µm, 1.2 µm, 1.2 µm, 1.3 µm, 1.4 µm, 1.5 µm, 1.6 µm, 1.7 µm, 1.8µm, 1.9 µm or 2 µm. It is particularly preferred that the substrate has a thickness of about 500 nm ± 50 nm.

Agarose is used as polymerizing agent, in particular agarose Type IX-A, Ultra-low Gelling Temperature.

The agarose has a concentration of 0.01% - 0.8% in solution, preferably in water.

In further preferred embodiments, at least two liposomes are in contact with the non-continuous substrate, more preferably all liposomes are in contact with the non-continuous substrate. In preferred embodiments, the liposomes are in direct contact with the substrate, i.e. no further layer or molecule mediates the contact between the liposomes and the substrate. Preferably the liposomes are linked to the substrate whilst they are formed, more preferably without requiring any anchor layer or molecule.

In further preferred embodiments, the liposomes are not surrounded by any substrate or matrix, i.e. preferably the liposomes are only in contact with the substrate or substrate layer at a minimal contact point.

The embodiments outlined above provide inter alia the following advantages: (i) it is not required to used any modified lipid for positioning liposomes, (ii) the liposomes are formed inside the chip, (iii) any achor layer is not required, since the liposomes are linked to the substrate while they are formed.

The at least two liposomes may comprise identical or different lipids and/or lipid compositions. Lipids may be selected from the group consisting of glycerolipids, glycerophospholipids, sphingolipids, saccharolipids, polyketides, prenol lipids, fatty acyls, and sterol lipids. Lipid compositions may comprise any number or mixture of lipids. Thus, a lipid composition may comprise a mixture of 2, 3, 4, 5, or more different lipids.

The size of the at least two liposomes may be identical or different. In preferred embodiments, the diameter of the liposome(s) is between 1 and 300 µm, i.e. 1, 2, 3, 4, 5, 10, 15, 20, 25, 50, 75, 100, 150, 200, 250, or 300 µm. The size of the liposome may be adjusted by increasing or decreasing the thickness and/or the concentration of the agarose. Thus, by increasing the thickness and/or concentration of the agarose, the size of the liposomes increases and by decreasing the thickness and/ or concentration of the substrate also the size of the liposome decreases. For instance, at a substrate concentration of 0.1% agarose, the liposome diameter may be between 4 and 5 µm, preferably about 4.5 µm, whereas at a substrate concentration of 0.4% agarose, the liposome diameter may be between 7 and 10 µm, preferably about 8 to 9 µm, most preferably about 8.3 µm, and where at a substrate concentration of 0.8% agarose, the liposome diameter may be between 11 and 17 µm, preferably about 13 to 15 µm, most preferably about 14.3 µm, further at a substrate concentration of 1.2% agarose, the liposome diameter may be between 18 and 28 µm, preferably about 23 to 24 µm, most preferably about 23.5 µm. Also for an increased or decreased substrate thickness a correlation to the size of the liposomes may be observed.

In further embodiments, the liposome array may be divided into two or more individual areas of liposomes which are preferably fluidly separated from each other by separating barriers. Preferably, the separating barrier consists of a material selected from the group consisting of a polymer, glass, and ceramic. In more preferred embodiments the separating barrier is made of a UV or heat sensitive polymer, preferably selected from the group consisting of thiolene based resin, ceramic, poly-(methyl methacrylate), and cyclic olefin copolymer. Particularly preferred is a UV sensitive thiolene based resin. In further preferred embodiments, the at least two separate areas of liposomes comprise identical or different lipids and/or lipid compositions which are preferably each independently selected from the group consisting of glycerolipids, glycerophospholipids, sphingolipids, saccharolipids, polyketides, prenol lipids, fatty acyls and sterol lipids. Lipid compositions may comprise any number or mixture of lipids. Thus, a lipid composition may comprise a mixture of 2, 3, 4, 5, or more different lipids.

In a second aspect, the present invention provides a liposome array comprising a carrier layer, a layer of substrate on the surface of the carrier layer and at least two separate areas of liposomes on its surface. Preferably in such liposome array, independently each of the at least two separate areas of liposomes has a surface of less than 1 mm², i.e. less than 1 mm², less than 0.99 mm², less than 0.98 mm², less than 0.97 mm², less than 0.96 mm², less than 0.95 mm², less than 0.94 mm², less than 0.93 mm², less than 0.92 mm², less than 0.91 mm², less than 0.90 mm², less than 0.85 mm², less than 0.80 mm², less than 0.75 mm², less than 0.70 mm², less than 0.65 mm², less than 0.60 mm², less than 0.55 mm², less than 0.50 mm², less than 0.45 mm², less than 0.40 mm², less than 0.35 mm², less than 0.30 mm², less than 0.25 mm², less than 0.20 mm², less than 0.15 mm², less than 0.10 mm², less than 0.5 mm², less than 0.25 mm², less than 0.2 mm², less than 0.1 mm², less than 0.05 mm², or even less. In particularly preferred embodiments independently each of the at least two separate areas of liposomes has a surface of about 0.50 mm².

The distance between adjacent areas of the liposome array of the second aspect is preferably between 10 µm to 5,000 µm. Thus, it is preferred that the distance between two areas adjacent is 10 µm, 20 µm, 30 µm, 40 µm, 50 µm, 60 µm, 70 µm, 80 µm, 90 µm, 100 µm, 110 µm, 120 µm, 130 µm, 140 µm, 150 µm, 160 µm, 170 µm, 180 µm, 190 µm, 200 µm, 250 µm, 300 µm, 350 µm, 400 µm, 450 µm, 500 µm, 550 µm, 600 µm, 650 µm, 700 µm, 750 µm, 800 µm, 850 µm, 900 µm, 950 µm, 1,000 µm, 1,100 µm, 1,200 µm, 1,300 µm, 1,400 µm, 1,500 µm, 1,600 µm, 1,700 µm, 1,800 µm, 1,900 µm, 2,000 µm, 2,100 µm, 2,200 µm, 2,300 µm, 2,400 µm, 2,500 µm, 2,600 µm, 2,700 µm, 2,800 µm, 2,900 µm, 3,000 µm, 3,100 µm, 3,200 µm, 3,300 µm, 3,400 µm, 3,500 µm, 3,600 µm, 3,700 µm, 3,800 µm, 3,900 µm, 4,000 µm, 4,100 µm, 4,200 µm, 4,300 µm, 4,400 µm, 4,500 µm, 4,600 µm, 4,700 µm, 4,800 µm, 4,900 µm, or 5,000 µm. In particularly preferred embodiments the distance between two areas adjacent is between 50 µm and 3000 µm, more preferably between 100 µm and 1000 µm, most preferably about 500 µm.

In preferred embodiments, each area of liposomes comprises at least 1 liposome. Each area of liposomes may also comprise more than 1 liposome, i.e. 2, 3, 4, 5, 10, 50, 100, 1,000, 10,000, 100,000, 1,000,000, or more liposomes. Preferably, at least one liposome is in contact with the substrate, more preferably all liposomes are in contact with the substrate. In preferred embodiments, the liposomes are in direct contact with the substrate, i.e. no further layer or different molecule mediates the contact between the liposomes and the substrate. Preferably the liposomes are linked to the substrate whilst they are formed, more preferably without requiring any anchor layer or molecule. In further preferred embodiments, the liposomes are not surrounded by any substrate or matrix, i.e. preferably the liposomes are only in contact with the substrate or substrate layer at a minimal contact point.

It is further preferred that the areas of liposomes are fluidly separated from each other by separating barriers. Preferably the separating barrier consists of a material selected from the group consisting of a polymer, glass, and ceramic. In more preferred embodiments the separating barrier is made of a UV or heat sensitive polymer, preferably selected from the group consisting of thiolene based resin, ceramic, poly-(methyl methacrylate), and cyclic olefin copolymer. Particularly preferred is a UV sensitive thiolene based resin.

In further preferred embodiments of the second aspect, at least two separate areas of liposomes comprise identical or different lipids and/or lipid compositions which are preferably each independently selected from the group consisting of glycerolipids, glycerophospholipids, sphingolipids, saccharolipids, polyketides, prenol lipids, fatty acyls and sterol lipids. Lipid compositions may comprise any number or mixture of lipids. Thus, a lipid composition may comprise a mixture of 2, 3, 4, 5, or more different lipids.

The size of the liposomes in each area of liposomes may be identical or different. In preferred embodiments, the diameter of the liposome(s) is between 1 and 300 µm i.e. 1, 2, 3, 4, 5, 10, 15, 20, 25, 50, 75, 100, 150, 200, 250, or 300 µm. The size of the liposome may be adjusted by increasing or decreasing the thickness and/or the concentration of the agarose substrate. Thus, by increasing the thickness and/ or concentration of the agarose substrate, the size of the liposomes increases and by decreasing the thickness and/ or concentration of the substrate also the size of the liposome decreases. The carrier layer may be either transparent or opaque. In preferred embodiments of the second aspect of the present invention the carrier layer is transparent. The carrier may be made of any suitable material, preferably selected from the group consisting of glass, silicon, polymer, ceramic, and plastic. In particularly preferred embodiments, the carrier layer is made of glass.

In further preferred embodiments of the second aspect of the present invention, the substrate is a continuous or a non-continuous substrate. In embodiments of a non-continuous substrate, all or at least a part of the non-continuous substrate is in the shape of one or more linear or curved stripes or ridges. These stripes or ridges preferably have a width of less than 10,000 µm, i.e. less than 10,000 µm, less than 9,000 µm, less than 8,000 µm, less than 7,000 µm, less than 6,000 µm, less than 5,000 µm, less than 4,000 µm, less than 3,000 µm, less than 2,000 µm, less than, 1,000 µm, less than 900 µm, less than 800 µm, less than 700 µm, less than 600 µm, less than 500 µm, less than 400 µm, less than 300 µm, less than 200 µm, less than 100 µm, less than 99 µm, less than 98 µm, less than 97 µm, less than 96 µm, less than 95 µm, less than 94 µm, less than 93 µm, less than 92 µm, less than 91 µm, less than 90 µm, less than 85 µm, less than 80 µm, less than 75 µm, less than 70 µm, less than 65 µm, less than 60 µm, less than 55 µm, less than 50 µm, less than 45 µm, less than 40 µm, less than 35 µm, less than 30 µm, less than 25 µm, less than 20 µm, less than 15 µm, less than 10 µm, less than 5 µm, less than 4 µm, less than 3 µm, less than 2 µm, less than 1 µm, or less than 0.5 µm. It is particularly preferred that the width of stripes or ridges is 1 µm to 100 µm, more preferably about 10 µm.

In further preferred embodiments, the distance between adjacent stripes or ridges is 0.1 µm or more, i.e. 0.1 µm, 0.2 µm, 0.3 µm, 0.4 µm, 0.5 µm, 0.6 µm, 0.7 µm, 0.8 µm, 0.9 µm, 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, 20 µm, 25 µm, 30 µm, 35 µm, 40 µm, 45 µm, 50 µm, 55 µm, 60 µm, 70 µm, 75 µm, 80 µm, 85 µm, 90 µm, 95 µm, 100 µm, or more. It is particularly preferred that the distance between adjacent stripes or ridges is between 5 µm and 15 µm, most preferably about 10 µm.

In preferred embodiments, the substrate may be patterned. Preferably the pattern has the shape selected from the group consisting of stripes, squares, rectangles, spots, circles and spirals. Typically, the pattern of the non-continuous substrate determines the position of the liposomes and the distance the liposomes have from each other.

In further preferred embodiments of the second aspect of the present invention, the thickness of the continuous and/or non-continuous substrate is between 1 nm to 2 µm, i.e. 1 nm, 2 nm, 3 nm, 4 nm, 5 nm, 6 nm, 7 nm, 8 nm, 9 nm, 10 nm, 15 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 200 nm, 300 nm, 400 nm, 500 nm, 600 nm, 700 nm, 800 nm, 900 nm, 1 µm, 1.1 µm, 1.2 µm, 1.2 µm, 1.3 µm, 1.4 µm, 1.5 µm, 1.6 µm, 1.7 µm, 1.8 µm, 1.9 µm or 2 µm. It is particularly preferred that the substrate has a thickness of about 500 nm ± 50 nm.

Agarose is used as polymerizing agent, in particular agarose Type IX-A, Ultra-low Gelling Temperature. The agarose has a concentration of 0.01% - 0.8% in solution, preferably in water.

In a third aspect, the present invention provides a dried liposome array comprising a liposome array according to the first or the second aspect of the present invention as specified herein above and below, wherein the solvent content in the liposome array is reduced to below 1 to 15 %, i.e. to less than 1%, less than 2%, less than 3%, less than 4%, less than 5%, less than 6%, less than 7%, less than 8%, less than 9%, less than 10%, less than 11%, less than 12%, less than 13%, less than 14%, or less than 15%.

In preferred embodiments, the solvent in which the lipids are resolved is reduced to less than 1%, i.e. less than 0.1%, less than 0.2%, less than 0.3%, less than 0.4%, less than 05%, less than 0.6%, less than 0.7%, less than 0.8%, less than 0.9%, or less than 1.0% . In further embodiments, the solvent in which the polymerizing agent, preferably the agarose, is resolved is reduced to between 1 - 15%, i.e. to 1%, 2%, 3%, 4%, 5%, 6%, 7%, 8%, 9%, 10%, 11%, 12%, 13%, 14%, or 15%.

In preferred embodiments the liposome array is dried via heat, airflow, or vacuum. For instance, the liposome array may be dried over night at room temperature or, to accelerate the drying process, the liposome array may be dried at increased temperature, such as but not limited to 37°C. Alternatively, the liposome array may be exposed to vacuum to accelerate the drying process.

In a fourth aspect the present invention provides a device comprising a liposome array according to aspects 1, 2 and/or a dried liposome array according to aspect 3 of the present invention as specified above or below. Preferably said device is a chip, more preferably a microchip. In preferred embodiments, the device further comprises means for the application of fluid to at least two separate liposomes in contact with the non-continuous substrate or to at least two areas of liposomes. In further preferred embodiments these means are microfluidic means, preferably selected from the group consisting of one or more microchannels and/or one or more micropumps. The device may also comprise further means such as but not limited to seringe pump, perisaltic pump, and pressure driven pump.

In further embodiments, it is preferred that the device, preferably the microchip, is produced by bonding of the different reservoirs of the microchip to the carrier in order to prevent cross contamination of lipids/proteins between the different reservoirs. Preferably the bonding method comprises the following steps:
1. providing a carrier, preferably a glass carrier,
2. applying a layer of bonding material to the carrier, preferably a polymer such as but not limited to PDMS diluted in hexane and thiolene based resin,
3. pressing the reservoirs, preferably made of a polymer such as e.g. PDMS, on the layer of bonding material,
4. releasing the reservoirs,
5. pressing the reservoirs on the continuous or non-continuous substrate layer of a liposome array as described above or below, optionally comprising previously applied lipids,
6. bonding the reservoirs to the liposome array and leave to dry overnight and
7. keeping the assembled device under inert gas.

In a fifth aspect the present invention provides a method of producing the liposome array of any of aspects 1, 2 and/or 3 comprising the steps of (a) applying a non-continuous substrate or a continuous substrate layer to a carrier or carrier layer, preferably by rolling, spraying, deep-coating, inking, printing, or microfluidic patterning, and (b) applying a lipid and/or a lipid composition to the non-continuous substrate or to the substrate layer.

In embodiments wherein the liposome array has a non-continuous substrate layer, the non-continuous substrate layer is preferably applied to the carrier layer by microfluidic patterning, such as e.g. soft-lithography, comprising the following steps
1. attaching a mask such as but not limited to a PDMS, glass, ceramic, and/or plastic mold, to the carrier, preferably wherein the mask comprises microchannels which have the shape, width, height and distance desired for the pattern of the non-continuous,
2. filling the mask, preferably the microchannels, with substrate solution, preferably with agarose solution, preferably by injecting the substrate solution into the microchannals by capillarity,
3. leaving the substrate solution to dry, preferably overnight at room temperature and
4. removing the mask from the carrier, preferably without removing the substrate layer.

For instance, the microchannels comprised in the mask may be patterned in stripes wherein each ridge has a width of 10 µm and neighboring stripes are separated from each in a distance of 10 µm. It is however understood that any other pattern such as squares, rectangles, spots, circles and spirals may be used. It is also understood that any other measurements of the ridges may be used. Thus, the ridges may have a width of less than 10,000 µm, i.e. less than 10,000 µm, less than 9,000 µm, less than 8,000 µm, less than 7,000 µm, less than 6,000 µm, less than 5,000 µm, less than 4,000 µm, less than 3,000 µm, less than 2,000 µm, less than, 1,000 µm, less than 900 µm, less than 800 µm, less than 700 µm, less than 600 µm, less than 500 µm, less than 400 µm, less than 300 µm, less than 200 µm, less than 100 µm, less than 99 µm, less than 98 µm, less than 97 µm, less than 96 µm, less than 95 µm, less than 94 µm, less than 93 µm, less than 92 µm, less than 91 µm, less than 90 µm, less than 85 µm, less than 80 µm, less than 75 µm, less than 70 µm, less than 65 µm, less than 60 µm, less than 55 µm, less than 50 µm, less than 45 µm, less than 40 µm, less than 35 µm, less than 30 µm, less than 25 µm, less than 20 µm, less than 15 µm, less than 10 µm, less than 5 µm, less than 4 µm, less than 3 µm, less than 2 µm, less than 1 µm, or less than 0.5 µm. It is particularly preferred that the width of stripes or ridges is 1 µm to 100 µm, more preferably about 10 µm. Further the distance between adjacent stripes or ridges is 0.1 µm or more, i.e. 0.1 µm, 0.2 µm, 0.3 µm, 0.4 µm, 0.5 µm, 0.6 µm, 0.7 µm, 0.8 µm, 0.9 µm, 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, 20 µm, 25 µm, 30 µm, 35 µm, 40 µm, 45 µm, 50 µm, 55 µm, 60 µm, 70 µm, 75 µm, 80 µm, 85 µm, 90 µm, 95 µm, 100 µm, or more. It is particularly preferred that the distance between adjacent stripes or ridges is between 5 µm and 15 µm, most preferably about 10 µm. Typically, the pattern of the microchannels determines the pattern of the non-continuous substrate and thus, also determines the position of the liposomes and the distance the liposomes have from each other.

In preferred embodiments the lipid or lipid composition is spotted onto the non-continuous substrate or the substrate layer. In further embodiments the method of producing a liposome array comprises after step (a) and before step (b), step (aa) of applying separating barriers to the continuous or non-continuous substrate and carrier layer or the substrate layer. Preferably, step (aa) of applying separating barriers comprises the steps of (i) applying a mask to the non-continuous substrate and carrier layer or the substrate layer, (ii) applying a solidifiable liquid compound to the mask (iii) solidifying the liquid compound, preferably by exposing the compound to UV radiation and/or heat; and (iv) removing the mask such that the solidified compound remains attached to the non-continuous substrate and carrier layer or the substrate layer, preferably by peeling of the mask form the solidified compound.

In preferred embodiments, the mask applied in step (i) is made of a material selected from the group consisting of glass, polymer, and plastic. In preferred embodiments the mask is made of a polymer, more preferably of a siloxane polymer, most preferably of polydimethylsiloxane such as but not limited to Dow Corning Sylgard 184.

In further preferred embodiments, the solidifiable liquid compound applied in step (ii) to form the separating barriers is preferably selected from the group consisting of glass, polymer, plastic, and ceramic. In preferred embodiments the solidifiable liquid compound is a thiolene based resin such as but not limited to NOA81 (Norland Products).

In further preferred embodiments of the fifth aspect of the present invention, the method of producing a liposome array further comprises step (c) of applying a solvent or solvent composition to the non-continuous substrate and carrier layer or the substrate layer. In preferred embodiments the solvent or solvent composition is a physiological buffer selected from the group consisting of phosphate buffered saline (PBS), HEPES, and potassium based buffer (KCl). In preferred embodiments the liposomes are formed upon application of the solvent or solvent composition. It is particularly preferred that liposomes are formed without the application of further means, e.g. it is preferred that no electric current or electric field is applied in order to form the liposomes.

The carrier layer may be either transparent or opaque. In preferred embodiments of the fifth aspect of the present invention the carrier layer is transparent. The carrier may be made of any suitable material, preferably selected from the group consisting of glass, silicon, polymer, ceramic, and plastic. In particularly preferred embodiments, the carrier layer is made of glass.

Agarose is used as polymerizing agent, in particular agarose Type IX-A, Ultra-low Gelling Temperature. The agarose has a concentration of 0.01% - 0.8% in solution, preferably in water.

In further preferred embodiments, the thickness of the substrate is between 1 nm to 2 µm, i.e. 1 nm, 2 nm, 3 nm, 4 nm, 5 nm, 6 nm, 7 nm, 8 nm, 9 nm, 10 nm, 15 run, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 200 nm, 300 nm, 400 nm, 500 nm, 600 nm, 700 nm, 800 nm, 900 nm, 1 µm, 1.1 µm, 1.2 µm, 1.2 µm, 1.3 µm, 1.4 µm, 1.5 µm, 1.6 µm, 1.7 µm, 1.8µm, 1.9 µm or 2 µm. It is particularly preferred that the substrate has a thickness of about 500 nm ± 50 nm.

In a preferred embodiment of the fifth aspect the present invention provides a method of producing a dried liposome array comprising the step of (a) producing a liposome array according to the fifth aspect of the present invention as specified above or below; and (b) reducing the solvent content in the liposome array to below 1-15 %, i.e. to less than 1%, less than 2%, less than 3%, less than 4%, less than 5%, less than 6%, less than 7%, less than 8%, less than 9%, less than 10%, less than 11%, less than 12%, less than 13%, less than 14%, or less than 15%. In preferred embodiments, the solvent in which the lipids are resolved is reduced to less than 1%, i.e. less than 0.1%, less than 0.2%, less than 0.3%, less than 0.4%, less than 05%, less than 0.6%, less than 0.7%, less than 0.8%, less than 0.9%, or less than 1.0% . In further embodiments, the solvent in which the polymerizing agent, preferably the agarose, is resolved is reduced to between 1 - 15%, i.e. to 1%, 2%, 3%, 4%, 5%, 6%, 7%, 8%, 9%, 10%, 11%, 12%, 13%, 14%, or 15%. Preferably, the solvent content is reduced via the application of heat, airflow, vacuum. For instance, the liposome array may be dried over night at room temperature or, to accelerate the drying process, the liposome array may be dried at increased temperature, such as but not limited to 37°C. Alternatively, the liposome array may be exposed to vacuum to accelerate the drying process.

In a sixth aspect the present invention provides a method of studying lipid interactions comprising the step of (a) applying a sample of interest to the liposome(s) of the liposome array of aspect 1 or 2 or the device of aspect 4 as specified above and below. Preferably, the sample of interest is selected from the group consisting of a small molecule library, a tissue and a cellular extract, purified proteins and/or protein complexes. More preferably, the sample of interest is a small molecule library comprising pharmaceutically active small molecule compounds. In preferred embodiments the method of studying lipid interactions further comprises step (b) of analyzing the lipid interaction. Lipid interactions may be analyzed via any method know in the art. In preferred embodiments, the lipid interactions are analyzed via microscopic means, more preferably via fluorescence microscopy and/or spectroscopy.

In a seventh aspect the present invention provides a method of screening a library comprising the step of (a) applying a library to the liposome(s) of the liposome array of aspect 1 or 2 or the device of aspect 4 as specified above or below. In preferred embodiments, the library comprises small molecule compounds, preferably pharmaceutically active small molecule compounds. In further preferred embodiments the method of screening a library comprises further step (b) of identifying compound that interact or do not interact with the liposomes. Compounds may be identified via any method known in the art.

In an eight aspect the present invention provides a method of producing liposomes of a predetermined size comprising the steps of: (a) forming a substrate of a thickness between 1 nm to 2 µm; and (b) applying a lipid or a lipid composition to at least one surface area of the substrate. Thus, by varying the thickness and/or the concentration of the substrate, preferably the agarose, the size of the liposomes is altered. By increasing the thickness and/ or concentration of the substrate, preferably the agarose, the size of the liposomes increases and by decreasing the thickness and/ or concentration of the substrate also the size of the liposome decreases. For instance, at a substrate concentration, preferably an agarose concentration, of 0.1%, the liposome diameter may be between 4 and 5 µm, preferably about 4.5 µm, whereas at a substrate concentration, preferably an agarose concentration, of 0.4%, the liposome diameter may be between 7 and 10 µm, preferably about 8 to 9 µm, most preferably about 8.3 µm, and where at a substrate concentration, preferably an agarose concentration, of 0.8%, the liposome diameter maybe between 11 and 17 µm, preferably about 13 to 15 µm, most preferably about 14.3 µm, further at a substrate concentration preferably an agarose concentration, of 1.2 %, the liposome diameter may be between 18 and 28 µm, preferably about 23 to 24 µm, most preferably about 23.5 µm. Also for an increased or decreased agarose thickness a correlation to the size of the liposomes may be observed.

In a ninth aspect the present invention provides a method of aligning liposomes comprising the steps of: (a) forming a non-continuous substrate; (b) applying a lipid and/or a lipid composition to at least one surface area of the substrate; and (c) forming liposomes from the lipid and/or the lipid composition of step (b). Preferably the liposome(s) contact the non-continuous substrate.

In further preferred embodiments of the ninth aspect of the present invention the non-continuous substrate is patterned. Preferably the pattern has the shape selected from the group consisting of stripes, squares, rectangles spots, circles, and spirals. Typically, the pattern of the non-continuous substrate determines the position of the liposomes and the distance the liposomes have from each other. In preferred embodiments the non-continuous substrate is in the shape of one or more linear or curved stripes or ridges. These stripes or ridges preferably have a width of less than 10,000 µm, i.e. less than 10,000 µm, less than 9,000 µm, less than 8,000 µm, less than 7,000 µm, less than 6,000 µm, less than 5,000 µm, less than 4,000 µm, less than 3,000 µm, less than 2,000 µm, less than, 1,000 µm, less than 900 µm, less than 800 µm, less than 700 µm, less than 600 µm, less than 500 µm, less than 400 µm, less than 300 µm, less than 200 µm, less than 100 µm, less than 99 µm, less than 98 µm, less than 97 µm, less than 96 µm, less than 95 µm, less than 94 µm, less than 93 µm, less than 92 µm, less than
91 µm, less than 90 µm, less than 85 µm, less than 80 µm, less than 75 µm, less than 70 µm, less than 65 µm, less than 60 µm, less than 55 µm, less than 50 µm, less than 45 µm, less than 40 µm, less than 35 µm, less than 30 µm, less than 25 µm, less than 20 µm, less than 15 µm, less than 10 µm, less than 5 µm, less than 4 µm, less than 3 µm, less than 2 µm, less than 1 µm, or less than 0.5 µm. It is particularly preferred that the width of stripes or ridges is 1 µm to 100 µm, more preferably about 10 µm.

In further preferred embodiments, the distance between adjacent stripes or ridges is 0.1 µm or more, i.e. 0.1 µm, 0.2 µm, 0.3 µm, 0.4 µm, 0.5 µm, 0.6 µm, 0.7 µm, 0.8 µm, 0.9 µm, 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, 20 µm, 25 µm, 30 µm, 35 µm, 40 µm, 45 µm, 50 µm, 55 µm, 60 µm, 70 µm, 75 µm, 80 µm, 85 µm, 90 µm, 95 µm, 100 µm, or more. It is particularly preferred that the distance between adjacent stripes or ridges is between 5 µm and 15 µm, most preferably about 10 µm.

In preferred embodiments, the substrate may be patterned. Preferably the pattern has the shape selected from the group consisting of stripes, squares, rectangles, circles and spirals.

In further preferred embodiments of the second aspect of the present invention, the thickness of the continuous and/or non-continuous substrate is between 1 nm to 2 µm, i.e. 1 nm, 2 nm, 3 nm, 4 nm, 5 nm, 6 nm, 7 nm, 8 nm, 9 nm, 10 nm, 15 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 200 nm, 300 nm, 400 nm, 500 nm, 600 nm, 700 nm, 800 nm, 900 nm, 1 µm, 1.1 µm, 1.2 µm, 1.2 µm, 1.3 µm, 1.4 µm, 1.5 µm, 1.6 µm, 1.7 µm, 1.8µm, 1.9 µm or 2 µm. It is particularly preferred that the substrate has a thickness of about 500 nm ± 50 nm.

Agarose is used as polymerizing agent, in particular agarose Type IX-A, Ultra-low Gelling Temperature. The agarose has a concentration of 0.01% - 0.8% in solution, preferably in water.

In tenth aspect the present invention provides a method of improving the shelf life of a liposome array comprising the step of: (a) reducing the solvent content in a liposome array of aspect 1 or 2 as specified herein above and below, preferably to a solvent content below 1-15 %, i.e. to less than 1%, less than 2%, less than 3%, less than 4%, less than 5%, less than 6%, less than 7%, less than 8%, less than 9%, less than 10%, less than 11%, less than 12%, less than 13%, less than 14%, or less than 15%. In preferred embodiments, the solvent in which the lipids are resolved is reduced to less than 1%, i.e. less than 0.1%, less than 0.2%, less than 0.3%, less than 0.4%, less than 05%, less than 0.6%, less than 0.7%, less than 0.8%, less than 0.9%, or less than 1.0% . In further embodiments, the solvent in which the polymerizing agent, preferably the agarose, is resolved is reduced to between 1 - 15%, i.e. to 1%, 2%, 3%, 4%, 5%, 6%, 7%, 8%, 9%, 10%, 11%, 12%, 13%, 14%, or 15%. Preferably, the solvent content is reduced via the application of heat, airflow, vacuum. For instance, the liposome array may be dried over night at room temperature or, to accelerate the drying process, the liposome array may be dried at increased temperature, such as but not limited to 37°C. Alternatively, the liposome array may be exposed to vacuum to accelerate the drying process.

### Examples

### Example 1: Fabrication of liposome array

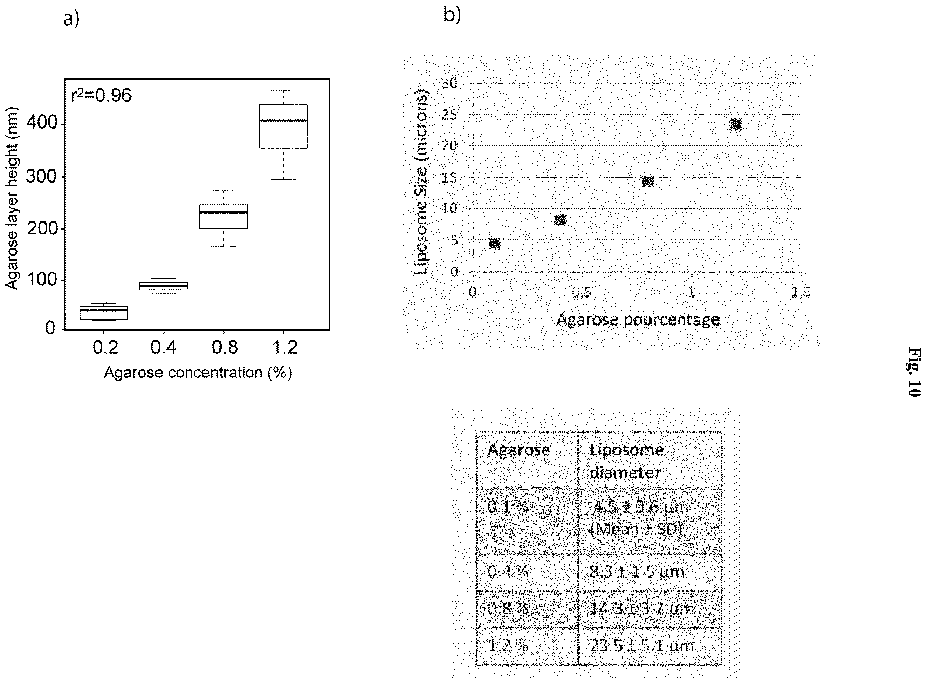

To produce a liposome array, 1% agarose Type IX-A, Ultra-low Gelling Temperature (Sigma, Germany) is dissolved in water. The solution is sprayed upon a glass microscope slide (30x45mm #1, Menzel-Glaser, Germany) serving as a carrier and left to dry overnight at room temperature resulting in a continuous layer of agarose fibres of 400 ± 30 nm on the glass slide (Fig.10(a)). In an alternative example, the agarose solution is patterned via microfluidic patterning onto the glass slide in the shape of stripes which have a thickness of 500 nm ± 50 nm and a width of 1000 nm ± 500 nm resulting in a non-continuous substrate layer. 80 nmol lipid solution comprising 400 nL of a 200 mM of lipid mixture POPC:DOPS with a ratio of 90:10 mol:mol in Chloroform:Methanol:water is now spotted (on a continuous substrate layer) or sprayed (on a non-continuous substrate layer) onto the agarose substrate layer with a spotter Camag Automatic TLC Sampler (Camag, Berlin, Germany) using non-contact mode.

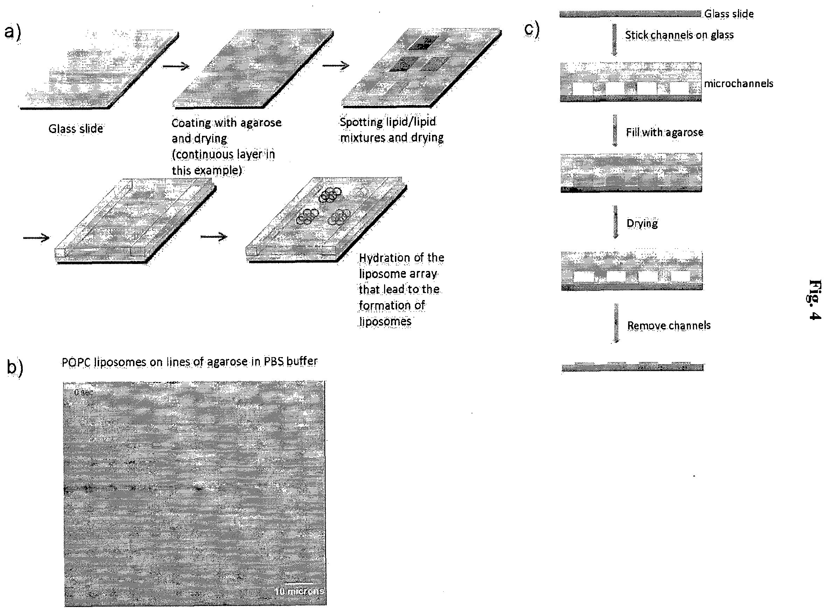

To create a non-continuous substrate layer microfluidic patterning is used wherein a PDMS mold with channels of 10 µm width separated from each other by 10 µm are obtained by soft-lithography methods. The channels are attached non-permanently to a glass slide. The agarose solution is injected in the channels by capillarity and dried at room temperature overnight. Then the PDMS mold is removed. Fig. 4(c) illustrates how to produce a non-continuous layer of agarose via microfluidic patterning.

### Example 2: Grid formation

To create a grid of several areas of liposomes, a protective mask of the siloxane polymer called Polydimethylsiloxane (Dow Corning, Sylgard 184) is placed on top of the agarose substrate of the liposome array described in Example 1 before the lipid solution is applied. Into the open spaces of the mask the UV sensitive thiolene based resin NOA81 (Norland Products) is applied. Upon exposure to UV radiation, the resin hardens now forming separating barriers between adjacent areas of liposomes. The protective mask is peeled of such that the separating barriers remain attached to the agarose layer. Into the now open areas of liposomes 80 nmol lipid solution comprising 400 nL of a 200 mM of lipid mixture POPC:DOPS with a ratio of 90:10 mol:mol in Chloroform:Methanol:water is now spotted (on a continuous substrate layer) or sprayed (on a non-continuous substrate layer) onto the agarose substrate layer with a spotter Camag Automatic TLC Sampler (Camag, Berlin, Germany) using non-contact mode.

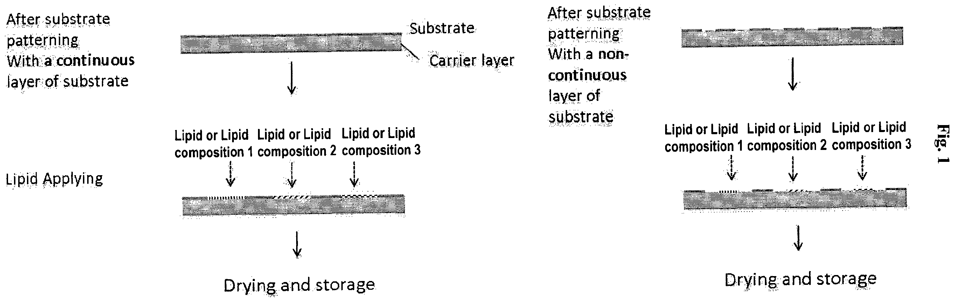

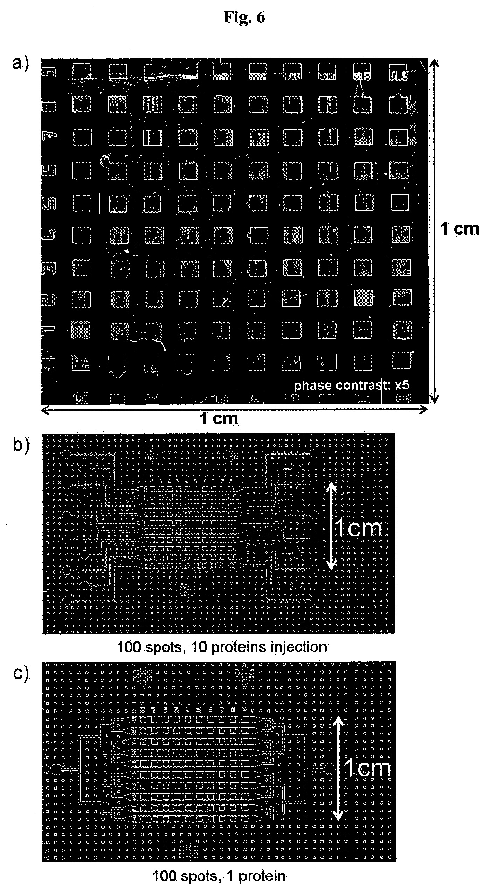

Figs. 1-4 show schematically how a liposome array as described in Example 1 and 2 is produced and Fig. 6(a) shows a phase contrast image of a grid of adjacent areas of liposomes is obtained.

### Example 3: Chip

In one example the liposome array as described in Examples 1 or 2 is integrated into a chip using glass as carrier layer. Microfluidic channels are connected to the liposome array to allow for the sample application.

Microfluidic channels made of PDMS by soft-lithography methods are bonded to the carrier layer recovered with the continuous or non-continuous substrate layer by exposing the chip for 1 min to plasma oxygen. In an alternative experiment, another bonding method comprising the steps of (i) creating a thin layer of PDMS prepolymer on a glass slide, (ii) transferring this thin layer to the microfluidic channel by stamping the channel onto the glass slide, (iii) bringing the channels in contact with substrate layer for bonding.

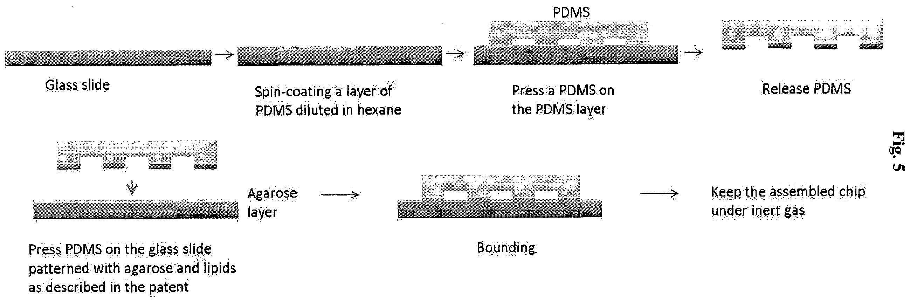

The bonding of a liposome array having a continuous layer of agarose to a microchip is illustrated schematically in Fig. 5 whilst Fig. 6 (b) and (c) provide examples of the liposome array integrated into a chip.

### Example 4: Liposome Formation

To obtain giant liposomes, the liposome array as described in Examples 1 and 2 is covered with the physiological buffer PBS resulting in the hydration and swelling of the film of agarose and the formation of liposomes attached to the surface of the agarose substrate layer.

Fig. 4(a) shows schematically how liposomes are formed and Fig. 4(b) provides a phase-contrast image of liposomes formed of 1-palmitoyl-2-oleoyl-sn-glycero-3-phosphocholine (POPC) on a non-continuous substrate layer of agarose.

### Example 5: Liposome Size and Spatial Placement

Rhodamin B- (Fig. 7a; positions 1, 3, 5) and BodipyFL-labeled (Fig. 7a; positions 2, 4) phospholipid mixtures of 89.9:10:0.1 1-palmitoyl-2-oleoyl-sn-glycero-3-phosphocholine (POPC):1,2-Dioleoyl-sn-glycero-3-phosphoserine (DOPS):Fluorescent lipid are sprayed on a non-continuous agarose substrate layer of an liposome array as described in Examples 1 and 2. Liposomes are formed as described in Example 4. Wild-field fluorescent microscopy of the liposomes is performed using Zeiss Axiovert 200.

Atto647-labeled (Fig. 7b; positions 2) and BodipyFL-labeled (Fig. 7b; positions 1 and 3) phospholipid mixtures of 89.9:10:0.1 1-palmitoyl-2-oleoyl-sn-glycero-3-phosphocholine (POPC):1,2-Dioleoyl-sn-glycero-3-phosphoserine (DOPS):Fluorescent lipid are sprayed on a continuous agarose substrate layer of an liposome array as described in Fig.3.

Fig. 7a and Fig.7b evidence that a liposome array can be formed without cross-contamination between the wells.

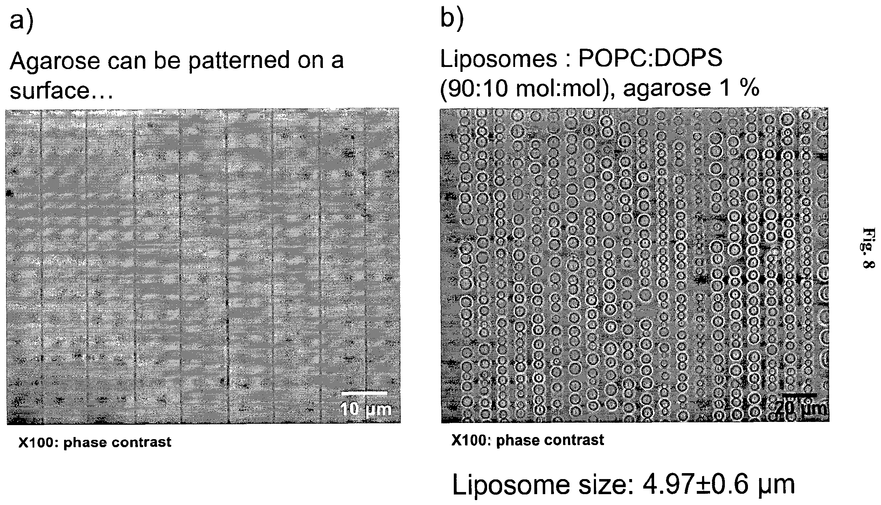

In Examples of a liposome array wherein the agarose is patterned, the position of the liposomes can be controlled in space and size. Fig. 8 (a) shows the phase contrast image of a non-continuous agarose substrate patterned in stripes. Upon application of a POPC:DOPS lipid mixture (90:10 mol:mol) liposomes are formed along the patterned agarose stripes (see Fig. 8 (b)). Thus, by varying the pattern of the non-continuous substrate layer, the position of the liposomes as well as their size can be controlled.

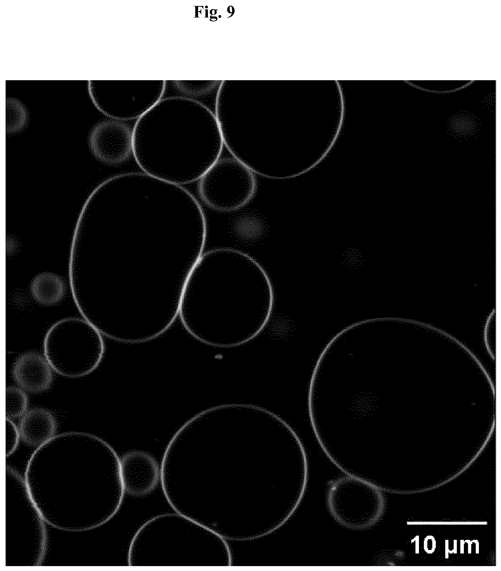

Fluorescently labels phospholipids POPC:DOPS:Phosphatidylethanolamine-Atto647 89.9:10:0.1 (mol:mol) are spotted or sprayed onto the agarose layer of the liposome array as described in Example 2. Liposomes are formed as described in Example 4. Confocal imaging of the liposomes is performed using a Leica SP5 microscope.
As shown in Fig. 9, the formed liposomes proved to be unilamelary.
To analyse the effect the concentration of the substrate has on the size of the formed liposomes, substrate comprising different concentrations, i.e. 0.1%, 0.4%, 0.8%, and 1.2 % of agarose were provided, and the size of the formed liposomes is measured. The experiments are performed 3 times. Fig. 10 illustrates the obtained results. Agarose layer height is proportional to the agarose concentration used to form a thin agarose layer (Fig. 10a). Each point represents an average of agarose height across 9 measurements (3 independent measurements on 3 different glass slides). A substrate concentration, here an agarose concentration, of 0.1% in solvent, here PBS, results in a liposome diameter of 4.5 ±0.6 µm, whereas a substrate concentration, here an agarose concentration, of 0.4% in solvent, here PBS, results in a liposome diameter of 8.3 ± 1.5 µm. A substrate concentration, here an agarose concentration, of 0.8% in solvent, here PBS, results in a liposome diameter of 14.3 ± 3.7 µm, and a substrate concentration, here an agarose concentration, of 1.2 % in solvent, here PBS, results in a liposome diameter of 23.5 ± 5.1 µm. Thus, Fig. 10 evidences that an increased concentration of substrate, here agarose, results in an increased size of liposomes.

### Example 6: AKT PH domain

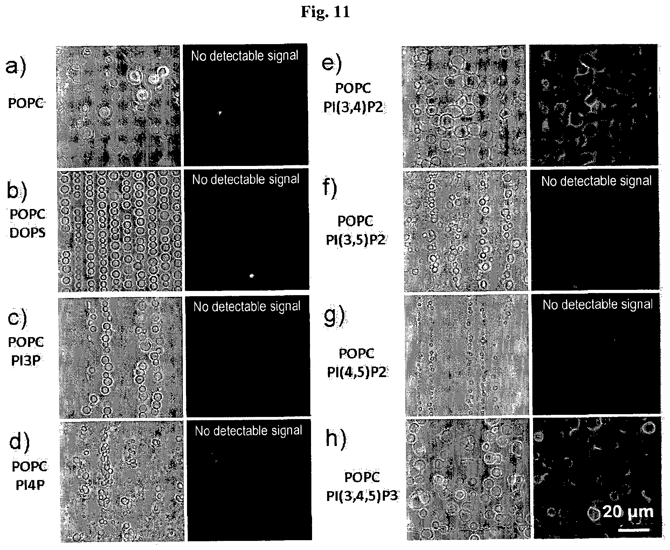

The Akt Pleckstrin Homology (PH) domain (positions 6 to 110) is known to specifically bind to PI-(3,4)-P2 and PI-(3,4,5)-P3 and is thus, used as a model system to evaluate the specificity and sensitivity of assays performed using the liposome array. The fluorescently labelled Akt PH domain is expressed in *Escherichia coli,* cells are lysed by sonication and after a centrifugation at 10000g, supernantant is recovered and injected in the microchip. Several liposome arrays each comprising liposomes formed from different lipids are produced. Liposomes either comprised POPC only, or a 93:3 mol:mol mixture of POPC:DOPS, POPC:PI3P, POPC:PI4P, POPC:PI(3,4)P2, POPC:PI(3,5)P2, POPC:PI(4,5)P2, POPC:PI(3,4,5)P3 (all of which are purchased from Avanti polar lipids) as shown in Fig. 11 (a) to (h), respectively.
*E.coli* lysate comprising the fluorescently labelled Akt PH domain is applied to the liposome array and incubated 30 minutes and washed with PBS buffer before imaging.

Wild-field fluorescent microscopy (Zeiss Axiovert 200) analysis of the different liposome arrays revealed that the Akt PH domain binds to liposomes comprising a mixture of POPC:PI(3,4)P2 or of POPC:PI(3,4,5)P3 confirming that the Akt PH domain only binds to PI-(3,4)-P2 but not to other lipids (see Fig. 11 (a) to (h)).

The sensitivity of the liposome array was assessed using a titration assay wherein different concentrations of *E.coli* expressed and His-tag purified AKT-PH domain were incubated with PS or PI(3,4)P2 comprising liposomes. After the hydration of the array with PBS, AKT PH domain purified is injected in the microchip and incubated 30 minutes. The protein is washed with PBS and liposomes are imaged with wild-field fluorescent microscopy (Zeiss Axiovert 200).

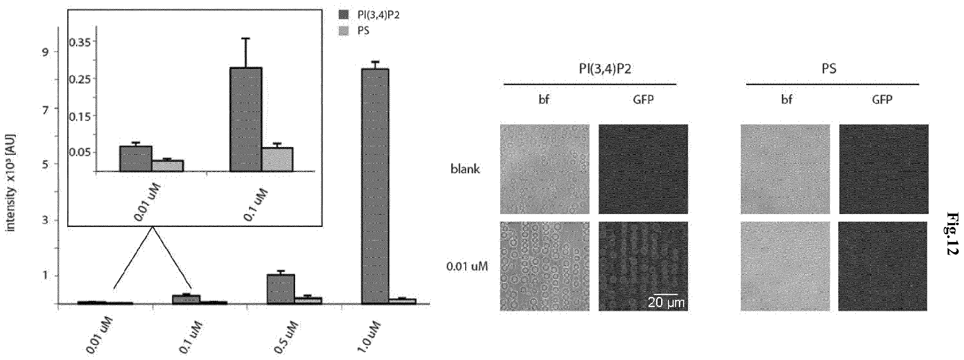

The results are illustrated in Fig. 12 evidencing that the sensitivity of the assay performed using the liposome array is at least equivalent to established methods.

### Example 7: Quality and Reproducibility of the liposome array

To evaluate the performance of the liposome array, the lipid-binding profile of a series of peripherally associated membrane proteins that cover six of the most prominent lipid-binding domains in eukaryotes was measured: the PH, PX, C1, C2, C2-like and PROPPIN domains (Fig. 14). The proteins were produced in *Escherichia coli* or human embryonic kidney 293 (HEK293) cells as GFP-tagged fusions. For each experiment, ∼12 µL of cell lysate (from 0.75 to 46,7 pmoles of protein) were loaded into individual chambers. Liposomes were produced upon hydration of the lipid-carrying thins agarose layers (TAL) with 4-(2-hydroxyethyl)-1-piperazineethanesulfonic acid (HEPES) buffer. The binding assay started with the introduction of the different GFP-tagged lipid-binding proteins into distinct chambers. Proteins and liposomes were allowed to interact during a 20 min incubation period, followed by washing to remove unbound material and imaging on an automated fluorescence microscopy platform. Fluorescence signal was analysed via a high-throughput image analysis pipeline (Fig. 13). The GFP-tagged proteins were expressed at significantly different levels and their membrane affinities varied over a broad range (Kd ranging from nM to µM). To capture this substantial physical diversity, the GFP signals at different exposure times (ranging from 5 to 1000 ms) were collected and subsequently removed the overexposed pixels. The Atto647 signal (PE-Atto647) was used to position the liposomes through image segmentation. Only GFP signals exactly matching the position of the liposomal membranes were further analyzed. Any background signal resulting from, for example, the nonspecific adsorption of proteins to the TAL or from protein aggregation was therefore efficiently filtered out. The Atto647 intensities reflect the actual surface area of membranes available for binding (i.e. liposome diameters and numbers). The GFP intensities indicate the number of GFPfusions bound to the liposomal membranes. A normalized binding intensity (NBI) - that is, the ratio of GFP to Atto647 fluorescence - was calculated to reflect the number of GFP-tagged proteins bound per membrane surface area. In other words, the NBIs are proportional to the amount of interacting proteins recruited to the liposomal membranes. The procedure is highly reproducible, as the NBIs measured with the same type of liposome in independent experiments correlated well (r²=0.88; Fig. 14b). The different LBD-containing proteins showed variations in their NBI depending on their specificity for particular lipids (Fig. 14c).

Indeed, the NBI profiles confirmed many known specificities, such as the interactions between the PX domain of p40phox and phosphatidylinositol 3-phosphate (PI(3)P); the Hsv2 protein (PROPPIN domain) and both PI(3)P and PI(3,5)P2; the C2-like domain of lactadherin (Lact-C2) and phosphatidylserine (PS)27; the PH domain of Akt1 and the product of the phosphatidylinositol- 3-kinase, PI(3,4)P2 and PS; and the PH domain of phospholipase Cδ1 and PI(4,5)P2. The assay is sensitive, as interactions with less than 1 pmole of protein could be measured, and quantitative, as the NBIs for an interacting protein-lipid pair were proportional to the amount of lipid and protein present in the assay (Fig. 14d). It also captures cooperative binding mechanisms such as the membrane recruitment of protein kinase C-δ (PkCδ, two C1 domains and one C2 domain) that requires both diacylglycerol (DAG) and PS, and the calcium-dependent recruitment of protein kinase C-y (Pkcy) to DAG/PS-containing membranes.

### Example 8: Disease-linked mutations can lead to different membrane-binding properties

Next it was assessed whether the liposome array can be used to measure discrete changes in binding affinities. Son-of-sevenless (Sos1) is a guanine nucleotide exchange factor with multiple lipid- and protein-binding domains that integrate complex regulatory inputs and thus control activation of the Ras GTPase (Fig. 15a). An E108K mutation in the amino-terminal histone-fold domain of Sos1 (Sos-HF) increases its affinity for phosphatidic acid (PA) and causes Noonan syndrome, a developmental disorder that includes heart malformation. In the assay, wild-type Sos-HF bound weakly and specifically to both PA and PI(4,5)P2 (Fig. 15b, c). The apparent affinity of Sos-HF containing the disease-associated E108K mutation was significantly increased for both PA and PI(4,5)P2 (the respective NBIs were two- and five-fold higher), and that increased binding of Sos1 to membrane lipids leads to Noonan Syndrome. The liposome array of the present invention therefore provides an integrated robust workflow that combines two powerful methods: rapid and efficient liposome production via a thin agarose layer and quantitative high throughput microscopy. The method delivers a protein-lipid interaction profile that is scalable to the proteome level. The liposome array allows the systematic mixing of lipids to probe for cooperative mechanisms, and proteins and/or protein complexes can be tested for their lipid-binding specificity.

### Example 9: PH domain lipid-binding properties

Pleckstrin homology (PH) domains are one of the most common domains in yeast as well as human genome (Lemmon et al., 2002). They are present in variety of proteins of different function and some of them are known to be implicated in human diseases including cancer, inflammation, cardiovascular and metabolic diseases (Bayascas et al., 2008; Carpten et al., 2007; Hussain et al., 2011; Lindhurst et al., 2011; Rawlings et al., 1993; Thomas et al., 1993). Despite being already tested in number of studies, the knowledge about PH domain ligand specificity is in most cases rather limited. PH domains are lipid binding domains generally considered to recognize phosphoinositides (Franke et al., 1997; Garcia et al., 1995; Klarlund et al., 1997; Lemmon et al., 1995; Salim et al., 1996). However, some are believed also to interact simultaneously with multiple lipids (Gallego et al., 2010; Maffucci and Falasca, 2001), but this hypothesis has never been tested in large-scale and on an unbiased manner.

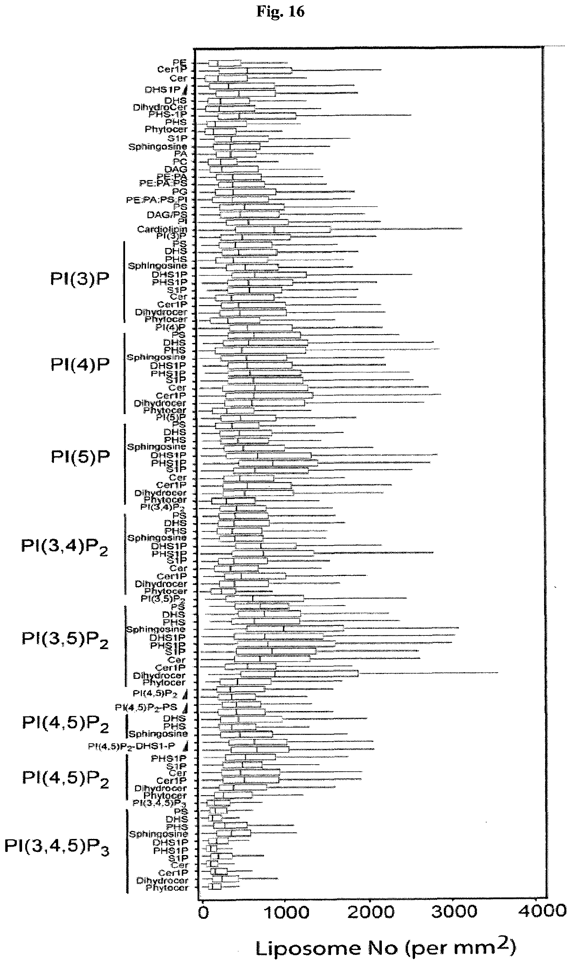

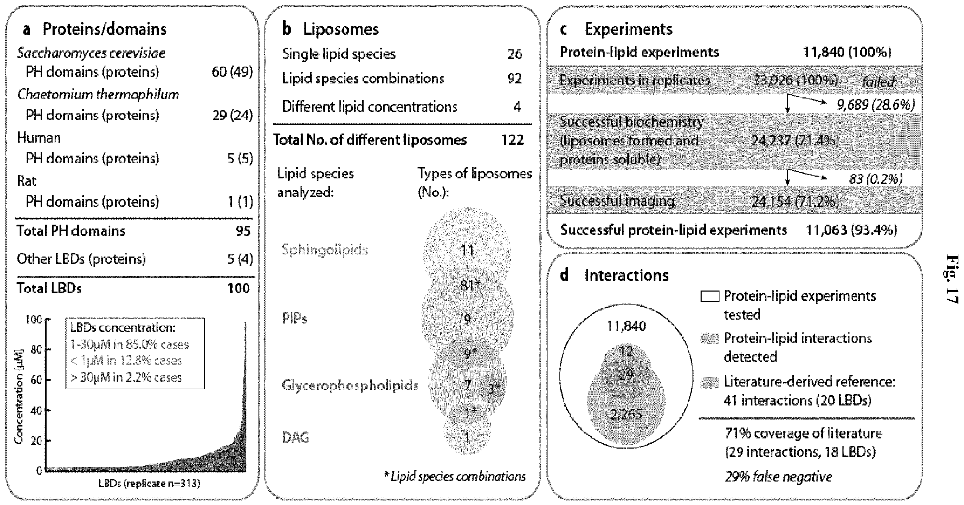

The aim of this screen is to systematically chart lipid-binding preferences of large group of PH domains for multiple lipid species and their combinations (Fig. 16 and Fig.17). A genome-wide screen has been designed including all PH domains present in *Saccharomyces cerevisiae* (number of domains = 60) and their orthologs in thermophilic fungus *Chaetomium thermophilum* (number of domains = 29). The screen also includes 6 mammalian PH domains and 5 other lipid-binding domains as controls. Altogether we probed 100 different lipid-binding domains, out of which 95 were PH domains, against 122 different types of liposomes (Fig.17a,b). This represents the largest protein-lipid screen so far consisting of 11,840 different protein-lipid experiments (Fig17c).

A liposome-array is fabricated following procedure described in Fig. 3 and bonded to a microfluidic device following procedure described in Fig. 5. Interactions are analysed following procedure described in Fig. 13.

In the screen 11,840 unique protein-lipid experiments were performed. Each experiment was tested in multiple replicates (3 replicates in vast majority of cases) which give a dataset of 33,926 experiments in total. Overall, 29% (9,772) of collected data was removed due to unsuccessful biochemistry (liposomes of low quality and/or protein precipitation) or imaging (unfocused images) (Fig. 17c). Further analysis was based on 24,154 (71%) experiments that covered 11,063 (93%) of unique protein-lipid experiments, with 77% (8,529) of them present in more than one replicate. The fact that 91% of 8,529 experiments with more than one replicate showed consistent result (reproducibly detected interaction/no interaction) indicates good reproducibility of the data.

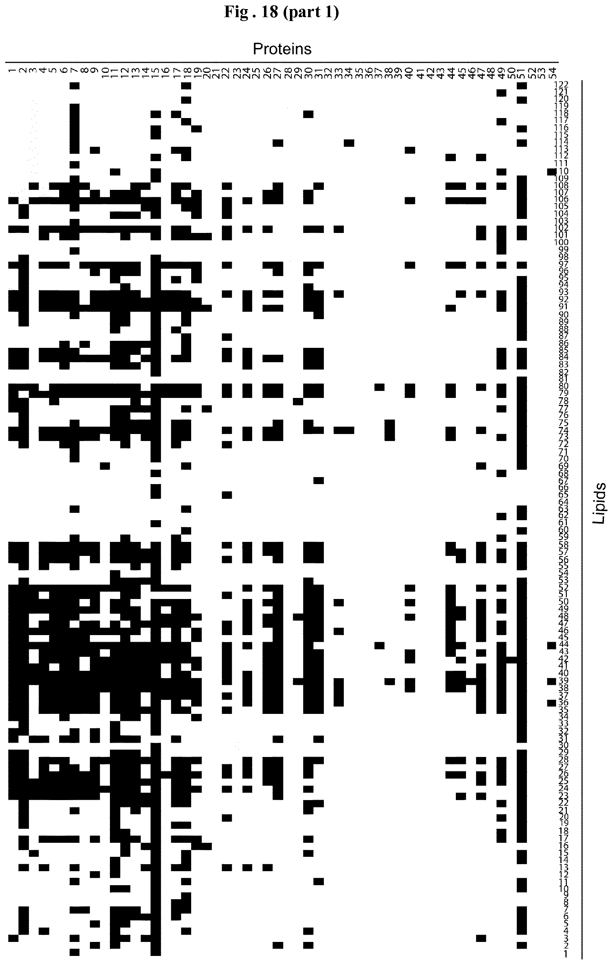

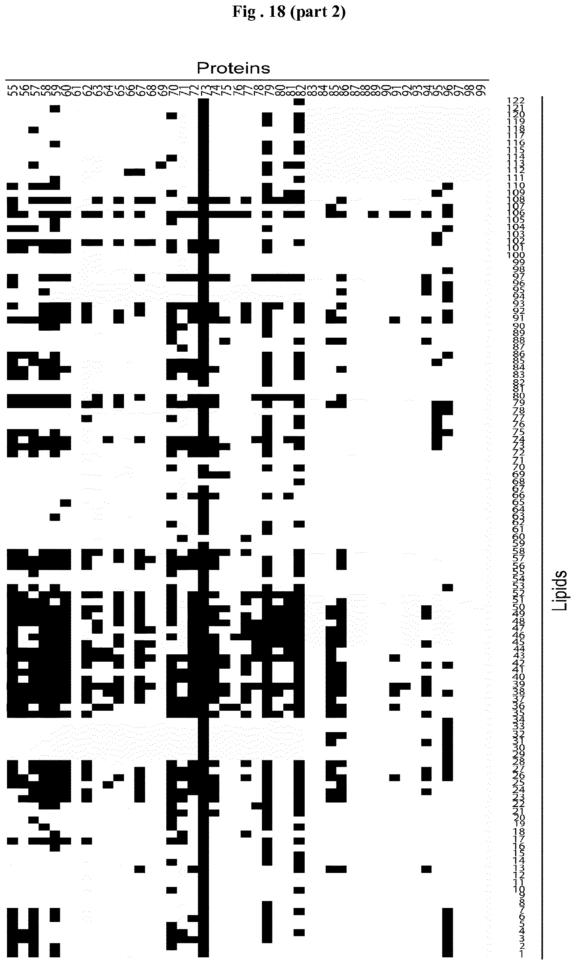

Altogether we detected 2,265 interactions (Fig. 17d and Fig. 18). For 76% (1,712) of them we have more than 1 replicate with consistent results in 72% (1,235) of the cases. To estimate the level of true interactions not captured in our dataset (false negative) the results were compared with interactions already know from the literature. We selected 41 well accepted interactions of 20 different lipid-binding domains as a reference set. All these interactions were tested in our screen and 29 of them (71%) were successfully captured, which indicates 29% false negative interactions (Fig. 17d)

## Claims

1. A liposome array comprising a carrier layer, layer of substrate on the surface of the carrier layer, and at least two separate areas of liposomes on its surface, wherein the substrate comprises agarose, wherein the agarose has a concentration of 0.01 to 0.8% in solution.

2. A liposome array comprising a carrier layer, a non-continuous substrate on the surface of the carrier layer and one or more liposomes in contact with the non-continuous substrate, wherein the substrate comprises agarose, wherein the agarose has a concentration of 0.01 to 0.8% in solution.

3. The liposome array according to any of claim 1 to 2, wherein the surface of the substrate is patterned, preferably in stripes, squares, rectangles, circles and/or spirals.

4. The liposome array according to any of claims 1 to 3, wherein at least two separate areas of liposomes comprise identical or different lipids and/or lipid compositions.

5. The liposome array according to any of claims 1 to 4, wherein the liposomes or areas of liposomes are fluidly separated from each other by separating barriers.

6. The liposome array of any of claims 1 to 5, wherein the diameter of the liposome(s) is between 1 and 300 µm.

7. A dried liposome array comprising a liposome array according to any of claims 1 to 6, wherein the solvent content in the liposome array is reduced to below 1 to 15 %.

8. A device comprising a liposome array according to any of claims 1 to 6 or a dried liposome array according to claim 7.

9. A method of producing the liposome array of any of claims 1 to 6 comprising the steps of
(a) applying a non-continuous substrate or a substrate layer to a carrier layer, preferably by rolling, spraying, deep-coating, inking, printing, or microfluidic patterning, wherein the substrate comprises agarose, wherein the agarose has a concentration of 0.01 to 0.8% in solution, and
(b) applying a lipid and/or a lipid composition to the non-continuous substrate or to at least two separate areas of the substrate.

10. The method of claim 9, further comprising the step of
(c) reducing the solvent content in the liposome array to below 1-15 %.

11. A method of studying lipid interactions comprising the step of
(a) applying a sample of interest to the liposome(s) of the liposome array of any of claims 1 to 6 or the device according to claim 8.

12. A method of screening a library comprising the step of
(a) applying a library to the liposome(s) of the liposome array of any of claims 1 to 6 or the device according to claim 8.

13. The method of producing the liposome array of claim 9 comprising the steps of:
(a) forming a substrate of a thickness between 1 nm to 2 µm, the substrate comprising agarose, wherein the agarose has a concentration of 0.01 to 0.8% in solution; and
(b) applying a lipid or a lipid composition to at least one surface area of the substrate.

14. The method of producing the liposome array of claim 9 comprising the steps of:
(a) forming a non-continuous substrate comprising agarose, wherein the agarose has a concentration of 0.01 to 0.8% in solution; and
(b) applying a lipid and/or a lipid composition to at least one surface area of the substrate; and
(c) forming liposomes from the lipid and/or the lipid composition of step (b).

15. A method of improving the shelf life of a liposome array comprising the step of:
(a) reducing the solvent content in a liposome array according to any of claims 1 to 6, preferably to a solvent content below 1-15 %.

## Patentansprüche

1. Liposomenarray umfassend eine Trägerschicht, eine Substratschicht auf der Oberfläche der Trägerschicht und mindestens zwei getrennte Bereiche von Liposomen auf ihrer Oberfläche, wobei das Substrat Agarose umfasst, wobei die Agarose eine Konzentration von 0,01 bis 0,8 % in Lösung aufweist.

2. Liposomenarray umfassend eine Trägerschicht, ein nicht-kontinuierliches Substrat auf der Oberfläche der Trägerschicht und ein oder mehrere Liposomen in Kontakt mit dem nicht-kontinuierlichen Substrat, wobei das Substrat Agarose umfasst, wobei die Agarose eine Konzentration von 0,01 bis 0,8 % in Lösung aufweist.

3. Liposomenarray nach einem der Ansprüche 1 bis 2, wobei die Oberfläche des Substrats strukturiert ist, vorzugsweise in Streifen, Quadraten, Rechtecken, Kreisen und/oder Spiralen.

4. Liposomenarray nach einem der Ansprüche 1 bis 3, wobei mindestens zwei separate Bereiche von Liposomen identische oder unterschiedliche Lipide und/oder Lipidzusammensetzungen umfassen.

5. Liposomenarray nach einem der Ansprüche 1 bis 4, wobei die Liposomen oder Bereiche von Liposomen durch Trennbarrieren fluidisch voneinander getrennt sind.

6. Liposomenarray nach einem der Ansprüche 1 bis 5, wobei der Durchmesser des Liposoms (der Liposomen) zwischen 1 und 300 µm beträgt.

7. Getrockneter Liposomenarray, umfassend einen Liposomenarray nach einem der Ansprüche 1 bis 6, wobei der Lösungsmittelgehalt in dem Liposomenarray auf unter 1 bis 15 % reduziert ist.

8. Vorrichtung, umfassend einen Liposomenarray nach einem der Ansprüche 1 bis 6 oder einen getrockneten Liposomenarray nach Anspruch 7.

9. Verfahren zum Herstellen des Liposomenarrays nach einem der Ansprüche 1 bis 6, umfassend die Schritte:
(a) Aufbringen eines nicht-kontinuierlichen Substrats oder einer Substratschicht auf eine Trägerschicht, vorzugsweise durch Walzen, Sprühen, Tauchbeschichten, Einfärben, Drucken oder mikrofluidische Strukturierung, wobei das Substrat Agarose umfasst, wobei die Agarose eine Konzentration von 0,01 bis 0,8 % in Lösung aufweist, und
(b) Aufbringen eines Lipids und/oder Lipidzusammensetzung auf das nicht kontinuierliche Substrat oder auf mindestens zwei getrennte Bereiche des Substrats.

10. Verfahren nach Anspruch 9, ferner umfassend den Schritt (c)
Reduzieren des Lösungsmittelgehalts in dem Liposomenarray auf unter 1 bis 15 %.

11. Verfahren zur Untersuchung von Lipidwechselwirkungen, umfassend den Schritt
(a) Aufbringen einer Probe von Interesse auf das Liposom (die Liposomen) des Liposomenarrays nach einem der Ansprüche 1 bis 6 oder der Vorrichtung nach Anspruch 8.

12. Verfahren zum Screenen einer Bibliothek, umfassend den Schritt
(a) Anwenden einer Bibliothek auf das Liposom (die Liposomen) des Liposomenarrays nach einem der Ansprüche 1 bis 6 oder der Vorrichtung nach Anspruch 8.

13. Verfahren zum Herstellen des Liposomenarrays nach Anspruch 9, umfassend die Schritte:
(a) Bilden eines Substrats mit einer Dicke zwischen 1 nm bis 2 µm, wobei das Substrat Agarose umfasst, wobei die Agarose eine Konzentration von 0,01 bis 0,8 % in Lösung aufweist; und
(b) Auftragen eines Lipids oder einer Lipidzusammensetzung auf mindestens eine Oberfläche des Substrats.

14. Verfahren zur Herstellung des Liposomenarrays nach Anspruch 9, umfassend die Schritte:
(a) Bilden eines nicht-kontinuierlichen Substrats, umfassend Agarose, wobei die Agarose eine Konzentration von 0,01 bis 0,8 % in Lösung aufweist; und
(b) Aufbringen eines Lipids und/oder einer Lipidzusammensetzung auf mindestens eine Oberfläche des Substrats; und
(c) Bilden von Liposomen aus dem Lipid und/oder der Lipidzusammensetzung von Schritt (b).

15. Verfahren zur Verbesserung der Lagerbeständigkeit eines Liposomenarrays, umfassend den Schritt:
(a) Reduzieren des Lösungsmittelgehalts in einem Liposomenarray nach einem der Ansprüche 1 bis 6, bevorzugt auf einen Lösungsmittelgehalt unter 1 bis 15 %.

## Revendications

1. Réseau à liposomes, comprenant une couche de support, une couche de substrat disposée sur la surface de la couche de support, et au moins deux régions à liposomes séparées sur sa surface, dans lequel le substrat comprend de l'agarose, et l'agarose présente une concentration de 0,01 à 0,8 % en solution.

2. Réseau à liposomes, comprenant une couche de support, un substrat non-continu disposé sur la surface de la couche de support, et un ou plusieurs liposome(s) en contact avec le substrat non-continu, dans lequel le substrat comprend de l'agarose, et l'agarose présente une concentration de 0,01 à 0,8 % en solution.

3. Réseau à liposomes conforme à l'une des revendications 1 à 2, dans lequel la surface du substrat porte un motif, de préférence en bandes, en carrés, en rectangles, en ronds et/ou en spirales.

4. Réseau à liposomes conforme à l'une des revendications 1 à 3, dans lequel au moins deux régions à liposomes séparées comprennent des lipides et/ou compositions de lipides identiques ou différent(e)s.

5. Réseau à liposomes conforme à l'une des revendications 1 à 4, dans lequel les liposomes ou les régions à liposomes sont séparées les un(e)s des autres de manière fluide par des barrières de séparation.

6. Réseau à liposomes conforme à l'une des revendications 1 à 5, dans lequel le diamètre du ou des liposome(s) vaut de 1 à 300 µm.

7. Réseau à liposomes séché, comprenant un réseau à liposomes conforme à l'une des revendications 1 à 6, dans lequel la teneur en solvant du réseau de liposomes est réduite à une valeur de moins de 1 % à 15 %.

8. Dispositif comprenant un réseau à liposomes conforme à l'une des revendications 1 à 6, ou un réseau à liposomes séché conforme à la revendication 7.

9. Procédé de production d'un réseau à liposomes conforme à l'une des revendications 1 à 6, comportant les étapes suivantes :
a) appliquer un substrat non-continu ou une couche de substrat sur une couche de support, de préférence par roulage, pulvérisation, immersion, encrage, impression, ou formation de motifs par voie microfluidique, étant entendu que le substrat comprend de l'agarose, et que l'agarose présente une concentration de 0,01 à 0,8 % en solution,
b) et appliquer un lipide et/ou une composition de lipides sur le substrat non-continu ou sur au moins deux régions séparées du substrat.

10. Procédé conforme à la revendication 9, qui comprend en outre l'étape suivante :
c) réduire la teneur en solvant du réseau à liposomes à une valeur de moins de 1 % à 15 %.

11. Procédé d'étude d'interactions de lipides, comprenant l'étape suivante :
a) appliquer un échantillon intéressant au(x) liposome(s) d'un réseau à liposomes conforme à l'une des revendications 1 à 6 ou d'un dispositif conforme à la revendication 8.

12. Procédé de criblage d'une bibliothèque, comprenant l'étape suivante :
a) appliquer une bibliothèque au(x) liposome(s) d'un réseau à liposomes conforme à l'une des revendications 1 à 6 ou d'un dispositif conforme à la revendication 8.

13. Procédé de production d'un réseau à liposomes, conforme à la revendication 9, comportant les étapes suivantes :
a) former un substrat d'épaisseur comprise entre 1 nm et 2 µm, étant entendu que le substrat comprend de l'agarose, et que l'agarose présente une concentration de 0,01 à 0,8 % en solution,
b) et appliquer un lipide ou une composition de lipides sur au moins une région de la surface du substrat.

14. Procédé de production d'un réseau à liposomes, conforme à la revendication 9, comportant les étapes suivantes :
a) former un substrat non-continu comprenant de l'agarose, étant entendu que l'agarose présente une concentration de 0,01 à 0,8 % en solution,
b) appliquer un lipide et/ou une composition de lipides sur au moins une région de la surface du substrat,
c) et former des liposomes à partir du lipide et/ou de la composition de lipides de l'étape (b).

15. Procédé permettant d'améliorer la durée de conservation d'un réseau à liposomes, comportant l'étape suivante :
a) réduire la teneur en solvant d'un réseau à liposomes conforme à l'une des revendications 1 à 6, de préférence jusqu'à une teneur en solvant de moins de 1 % à 15 %.
